# EUROPEAN PATENT APPLICATION

(11) **EP 3 590 914 A1**
(43) Date of publication of application: **08.01.2020**
(21) Application number: 18305893.2
(22) Date of filing: 05.07.2018
(51) Int. Cl.: C07C 39/23, C07C 43/205, C07C 49/603, C07C 255/07, C07F 7/08, C09B 11/02, C09B 11/06

(54) **MOLECULAR ORGANIC MATERIALS DERIVED FROM ANIONIC PARA-QUINONE METHIDE DYES AND THE LIKE, WITH SPECIAL OPTICAL EFFECTS**

(71) Applicant: Centre National de la Recherche Scientifique - CNRS, 75016 Paris (FR); Université Paris Diderot Paris 7, 75013 Paris (FR); Sorbonne Université, 75006 Paris (FR)
(72) Inventor: LAINE, Philippe, 75014 Paris (FR); POULARD, Laurélie, 93600 Aulnay sous Bois (FR); DUPEYRE, Grégory, 94510 La Queue en Brie (FR); MARVAUD, Valérie, 75014 Paris (FR)
(74) Representative: A.P.I. Conseil

(57) **Abstract**

The present invention relates to a compound of the following formula (I):

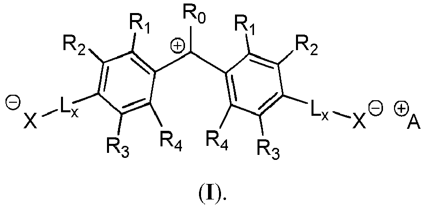

The invention also relates to uses thereof as dye or pigment, notably as a luster pigment, and to a reflective or photonic or nanophotonic or optoelectronic device and to a metal-like reflective coating or a metal-like liquid film, comprising a compound of the invention.

## Description

### FIELD OF THE INVENTION

The present invention relates to sterically congested, V-shaped anionic para-quinone methide derivatives (more particularly 4-(4-hydroxybenzylidene)cyclohexa-2,5-dienone) and their thio counterparts (that encompasses compounds bearing an extra aryl motif on methylidene bridge position, comprised of only two main "electroactive arms" so that their overall key V-shaped topology is preserved), as well as their use as molecular chromophores of interest as such (e.g. dye molecules) and as components of pigments displaying special optical effects or of organic metal-like liquid films.

Indeed, said anionic *para*-(thio)quinone methide derivatives of tunable electronic features can serve as the building blocks of organized organic molecular materials exhibiting special optical properties including (1) metal-like reflection or (2) iridescence or pearl-luster effects, that are referred to as special optical effects that respectively originate from interaction of light with the material surface, in the former case (1), or with the bulk material, in the latter case (2).

### BACKGROUND OF THE INVENTION

Quinone methides, and para-quinone methides in particular, are quite interesting synthons that have been proposed as intermediates in numerous biosynthetic pathways (e.g. catecholamine metabolism [Sugumaran, M. Int J Mol Sci., 2016, 17 (9), 1576], insect cuticle chemistry [Andersen, S.O. Insect Biochem. Mol. Biol., 2010, 40, 166-178], wood lignin chemistry [Shevchenko et al. Russ. Chem. Rev., 1992, 61 (1), 105-131]), in the chemical reactivity of antitumour compounds (see [Fan et al. Chem. Res. Toxicol. 2000, 13, 45-52] and [Angle et al. J. Org. Chem., 1997, 62, 5884-5892]) and in many chemical mechanisms involving their Michael acceptor properties towards various nucleophiles (see [Raju et al. Tetrahedron Lett., 2004, 45, 7487-7489], [Chu et al. Angew. Chem. Int. Ed., 2013, 52, 9229-9233], [Reddy et al. Org. Lett., 2015, 17, 3390-3393] and [Gao et al. Eur. J. Org. Chem., 2016, 3006-3012]).

If resort to *para*-quinone methides is so widespread, either in Nature or in chemist's hands, it's because of its versatility. The high reactivity of this motif is linked to the strong dipolar character coming from its asymmetrical disubstitution (by either a carbonyl and a methylidene group). Whereas genuine quinone methides are too reactive to be isolated and often lead to complex mixtures, methylidene-substituted ones allow manipulation and have been advantageously used for intramolecular cyclization reactions for example (see [Angle et al. Tetrahedron Lett., 1989, 30 (10), 1193-1196] and [Angle et al. J. Org. Chem., 1994, 59, 6322-6337]). 2,6-Disubstitution imparts also considerably the stability of para-quinone methides and use of such a substitution pattern is a prerequisite to perform chemistry on such systems.

To go further, a more efficient way to stabilize a *para*-quinone methide compound consists in its grafting with a symmetrical hindered phenol, a perfect example of this strategy is the prototypical hydrogalvinoxyl, where hindered positions surrounding quinone/phenol functions are occupied by *tert*-butyl groups. Oxidation of hydrogalvinoxyl conducts to a radical (named galvinoxyl) which is so stable that it is even insensitive to the presence of oxygen, (see [Coppinger, G.M. J. Am. Chem. Soc., 1957, 79 (2), 501-502], [Kharasch et al. J. Org. Chem., 1957, 22 (11), 1439-1443], and [Steelink et al. J. Am. Chem. Soc., 1968, 4354-4361]). Indeed, due its symmetry (favoring full conjugation) and to the presence of bulky protecting alkyl motifs, this radical represents the parangon of stabilized molecular organic radicals, a reason why it has been extensively studied [Novak et al. Chem. Phys. Lett., 2005, 413, 351-355] and more and more incorporated in molecular materials [Mas-Torrent et al. Chem. Rev., 2012, 112, 2506-2527].

As a symmetrical highly conjugated zwitterion, a phenolate form of such a 4-(4-hydroxybenzylidene)cyclohexa-2,5-dienone represents a putative good candidate for design of very powerful chromophores. Nevertheless, very scarce literature has been published on such molecules (see [Steelink et al. Tetrahedron Lett., 1966, 1, 105-111] and [Bukharov et al. Russ. J. Gen. Chem., 1998, 68 (3), 429-434].

It's worth noticing that such anionic quinone methide dyes are close to the family of fluorone dyes, a very important class of pH-sensitive fluorescent chromophores among which one can cite fluoresceins, eosins, "Greens" (e.g. Tokyo-, Magenta-, Pennsylvania-, Oregon-, Aarhus-) (see [Sun et al. J. Org. Chem., 1997, 62, 6469-6475], [Mottram et al. Org. Lett., 2006, 8 (4), 581-584] and [Zhang et al. J. Fluoresc., 2014, 24, 819-826].

The class of compounds here described deviates nevertheless significantly from these well-known dyes since combined absence of intercycle O-bridging or of a third cycle (in most case) and presence of a persubstituted pattern (either hindering the vicinity of the quinone/phenolate positions and of the methylidene bridge) confers them quite specific features.

Specular reflection of light is generally related to electronic conduction (surface plasmon). However, the development of metal-free, non-conductive, reflective organic molecular materials, relying on excitonic processes would pave the way for a wide variety of applications in a broad spectrum of areas: from reflectors or mirrors to photonics, paints and coatings (including niche implementations for which co-existence of electronic conduction and light reflection is detrimental), security inks, cosmetics, optoelectronics and laser technology, amongst others.

Special effect pigments are nano- or meso-particulate materials that give additional color effects, such as angular color dependence (iridescence, luster) or texture. Also named "luster pigments", these pigments are subdivided in 2 classes: metal effect pigments and pearl luster pigments (see [Special Effect Pigments, G. Pfaff, 2008, 2nd Rev. ed., Vincentz Network GmbH & Co. KG, Hannover/Germany] and [Metal Effect Pigments, Fundamentals and Applications, P. Wiβling, 2006, Vincentz Network GmbH & Co. KG, Hannover/Germany]).

With the exception of examples of "effect pigments" based on organic structures, amongst which so-called "photonic crystals" mostly encountered in Nature (e.g. guanine platelets isolated from fish scales and certain liquid crystals), the industry of "effect pigments" relies almost exclusively on metallic particles and purposely-structured inorganic materials (e.g. crystalline HgCl₂ platelets, lead, arsenic or bismuth salts, platelet-shaped PbHPO₄, mica-TiO₂ combination, basic lead carbonate, bismuth oxychloride, aluminum platelets coated with Fe₂O₃, metal oxide-coated synthetic mica, iron oxides mica, chromium(III) oxide mica, Al₂O₃ flakes, SiO₂ flakes, borosilicate flakes).

Most of these pigments suffer from environmental and/or durability issues: for instance, mercury, lead, arsenic and chromium are considered to be highly toxic.

Hence, there is a need for organic iridescent/pearlescent pigments, environmentally benign and durable.

Hitherto, by definition, "metallic effect pigment" is a subfamily of the "metallic pigment" group that includes "metal pigments" (consisting of pure metals or metal alloys) and either inorganic or organic color pigments possessing at least one metal atom in their formula. Here we propose to use purely organic molecular materials as "metallic effect pigments".

Although conductive polymers are known to display a metallic luster (see [Tanaka et al. Bull. Chem. Soc. Jpn. 1980, 53, 3430-3435], [Morikita et al. Adv. Mater. 2001, 13, 1862-1864] and [Yamamoto et al. Macromolecules 2003, 36, 4262-4267]), very few low molecular weight materials exhibit such a behavior and none is of any significance for industry. Only scarce examples can be cited from the literature, such as 1-aryl-2-[5-(tricyanoethenyl)-2-thienyl]pyrroles, (*E*)-5,5'-di(thiophen-2-yl)-3,3'-bi[thiophen-3(2*H*)-ylidene]-2,2'-diones, bis[4-(2-dimethylaminoethoxy)phenyl]diazene and a donor-acceptor molecule bearing two boron(III) diketonate moieties (see [Ogura et al. Org. Biomol. Chem. 2003, 1, 3845-3850], [Ogura et al. Tetrahedron 2006, 62, 2484-2491], [Evans et al. Org. Biomol. Chem. 2013, 11, 3871-3879], [Kondo et al. Langmuir 2014, 30, 4422-4426] and [Poon et al. Angew. Chem. Int. Ed. 2016, 128, 3711-3715]). Those compounds are resulting from serendipity rather than from a specific molecular design, and most often, they are not fully characterized, including from the standpoint of the rationalization of their unusual optical properties.

Noteworthy, however, is the reported example of a polymeric material based on polyvinyl alcohol (PVA) and doped with J-aggregates of TDBC (5,6-dichloro-2-[[5,6-dichloro-1-ethyl-3-(4-sulphobutyl)-benzimidazol-2-ylidene]-propenyl]-1-ethyl-3-(4-sulphobutyl)benzimidazolium hydroxide, sodium salt), that displays metal-like optical properties (see [Gentile et al. Nano Lett. 2014, 14, 2339-2344] and [Gentile et al. J. Opt. 2016, 18, 015001]).

Therefore, there is a need for reflective organic materials alternative to inorganic and metal-based compounds. For this purpose, the present invention relies on the self-assembly of specifically-conceived chromophores that feature huge molar extinction coefficients and very sharp absorption bands.

In this context, there exists a strong interest in developing chromophoric building blocks featuring above-mentioned electronic features that are namely new stable anionic (thio)quinone methide derivatives of V-shaped topology. Indeed, on the one hand, the individual compounds are interesting by themselves, as highly efficient chromophores. On the other hand, these salts can self-assemble, allowing the formation of anisotropic organic supramolecular materials displaying "special optical effects", including liquid mirrors.

In the mirror liquid domain, mercury and gallium alloys have long been prevailing given their metallic features and low melting points. More recently, alternative strategies have been proposed, notably the MELLF (Metal-Like Liquid Films) procedure able to produce highly reflective silver films at the interface between two immiscible liquids by using simple chemical deposition techniques with a mixture composed of AgNO₃, a reducing agent and a surfactant (see [Yogev et al. J. Phys. Chem., 1988, 92, 5754-5760] and [Efrima, S. Crit. Rev. Surf. Chem. 1991, 1, 167-215]).

The MEELF technology is now well established (see [US6951398] and [Yen et al. ACS Appl. Mater. Interfaces, 2014, 6, 4292-4300]), so that it is considered, to date, as the best alternative to metal liquid films and not appealing simply for its optical properties but for numerous applications such as optoelectronics, sensing, catalysis, and surface-enhanced Raman spectroscopy [Lu et al. Chem. Mater., 2018, 30, 1989-1997].

The benefits of a liquid mirror, compared to a conventional solid one, are numerous: 1) access to a cheap very large reflective surface displaying a high numerical aperture (e. g. for astronomy, space debris observation, LIDAR systems) 2) easy access to a quasi-flawless reflective surface, easy to clean and able to self-healing, 3) access to a reflective surface easy to ship and assemble (e.g. in order to install a lunar telescope, see [Borra et al. Nature, 2007, 447, 979-981]), 4) remove of the perspective or parallax error produced by conventional lenses that requires a sensor with an optical aperture larger than the object itself (telecentric scanning lenses for increasing image quality and applications including metrology, gauging, CCD based measurement, or microlithography [Thibault *et al. SPIE,* 1997, *3100,* 206-213]).

Consequently, apart from applications yet attainable (even if more expensively) with solid mirrors, such a technology may address other needs. Liquid mirror may be shaped by rotation or by thermal or magnetic fields (see [Truong, L. et al. Appl. Opt., 2005, 44, 1595], [Déry et al. Chem. Mater., 2008, 20, 6420-6426] and [EP1361585]), or may enter in the composition of a reflective renewable fluid in a device submitted to highly damaging, extremely intense photon beams [US 20020135908].

Another of the most promising application is the electrical control of a metal nanoparticles-based reflecting surface at an interface between water and oil using the ITIES system (for Interface of Two Immiscible Electrolytic Solutions), that if controlled opens the way towards real-time electroswitchable smart mirrors for smart window applications, for example (see [Flatté et al. J. Phys. Chem. C, 2010, 114, 1735-1747] and [Montelongo et al. Nat. Mater., 2017, 16, 1127-1136]).

Due to the intrinsic anisotropy displayed by the molecules objects of the present invention, the decoration of their molecular core with long hydrophobic chains makes them behaving as amphiphilic to mesogenic (liquid crystalline) dyes (depending on the nature and rigidity of extension groups), that render them particularly well suited for the design of a novel class of organic-based metal-like liquid films. Moreover, the chromophoric nature of the molecules, that originates in a particularly efficient inner charge transfer (characterized by both a high molar extinction coefficient and a sharp absorption band) induce the self-assembly of films or layered surfaces displaying highly reflective properties without resort to any metal, paving the way to inexpensive, environmentally benign and durable liquid mirrors (moreover possibly electroswitchable, given the reducible character of the carbenium here described).

### SUMMARY OF THE INVENTION

The present invention relates thus to a compound of the following formula (**I**): 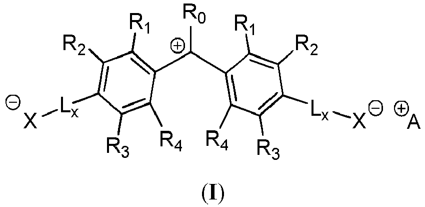 wherein:
▪ **X** represents an oxygen or a sulfur atom;
▪ **R₀** represents a hydrogen atom, a halogen atom, a (C₁-C₆)alkyl, (C₁-C₆)haloalkyl, aryl-(Ci-C₆)alkyl, heteroaryl-(C₁-C₆)alkyl, cycloalkyl, cycloalkyl-(C₁-C₆)alkyl, heterocycle, heterocycle-(C₁-C₆)alkyl, OR₅, SR₆, NR₇R₈, PR₉R₁₀, COR₁₁, CO₂R₁₂, CONR₁₃R₁₄, SO₂R₁₅, SO₃H, CN, NO₂, OCOR₁₆, OCO₂R₁₇, NR₁₈COR₁₉ or NR₂₀SO₂R₂₁ group, or is selected from the group consisting of: 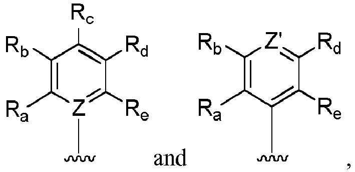 wherein:
   - Z represents C or N⁺A_{z}⁻ and Z' represents N or N⁺-R_{c}' A_{z}⁻, wherein
      A_{z}⁻ represents a monovalent organic or inorganic anion, and
      R_{c}' represents a (C₁-C₆)alkyl group,
   - Rₐ and Rₑ each represent, independently of each other, a hydrogen atom, a halogen atom, a (C₁-C₂₀)alkyl, (C₁-C₂₀)haloalkyl, aryl, heteroaryl, aryl-(C₁-C₆)alkyl, heteroaryl-(C₁-C₆)alkyl, cycloalkyl, cycloalkyl-(C₁-C₆)alkyl, heterocycle, heterocycle-(C₁-C₆)alkyl, OR₂₂ or SR₂₃ group, and
   - R_{b}, R_{c} and R_{d} each represent, independently of each other, a hydrogen atom, a halogen atom or a (C₁-C₂₀)alkyl, (C₁-C₂₀)haloalkyl, cycloalkyl, cycloalkyl-(C₁-C₆)alkyl, heterocycle, heterocycle-(C₁-C₆)alkyl, O(C₁-C₂₀)alkyl group;
▪ **R₁** represents a halogen atom, a (C₁-C₂₀)alkyl, (C₂-C₆)alkenyl, (C₂-C₆)alkynyl, (C₁-C₂₀)haloalkyl, aryl, heteroaryl, aryl-(C₁-C₆)alkyl, heteroaryl-(C₁-C₆)alkyl, cycloalkyl, cycloalkyl-(C₁-C₆)alkyl, heterocycle, heterocycle-(C₁-C₆)alkyl, (CH₂)ₘOR₂₄, (CH₂)_{m'}SR₂₅, OR₂₆, SR₂₇, NR₂₈R₂₉, PR₃₀R₃₁, COR₃₂, CO₂R₃₃, CONR₃₄R₃₅, OCOR₃₆, OCO₂R₃₇, NR₃₈COR₃₉, or NR₄₀SO₂R₄₁ group, wherein m and m' are, independently of each other, equal to 1, 2 or 3, preferably 1;
▪ **R₄** represents a halogen atom, a (C₁-C₂₀)alkyl, (C₂-C₆)alkenyl, (C₂-C₆)alkynyl, (C₁-C₂₀)haloalkyl, aryl, heteroaryl, aryl-(C₁-C₆)alkyl, heteroaryl-(C₁-C₆)alkyl, cycloalkyl, cycloalkyl-(C₁-C₆)alkyl, heterocycle, heterocycle-(C₁-C₆)alkyl, (CH₂)ₘOR₂₄, (CH₂)_{m'}SR₂₅, OR₂₆, SR₂₇, NR₂₈R₂₉, PR₃₀R₃₁, COR₃₂, CO₂R₃₃, CONR₃₄R₃₅, OCOR₃₆, OCO₂R₃₇, NR₃₈COR₃₉, or NR₄₀SO₂R₄₁ group, wherein m and m' are, independently of each other, equal to 1, 2 or 3, preferably 1;
   or both R₄ groups form together a bond or a chain selected from the group consisting of -C(R₄₂R₄₃)-, -(CH₂)ₙ-, -Si(R₄₄R₄₅)-, -(CH₂)ₚ-Y-(CH₂)_{q}-, and -Y-(CR₄₆R₄₇)ᵣ-Y'-, wherein:
   - Y and Y' each represent, independently of each other, O, S or NR₆₈,
   - n is equal to 2 or 3,
   - p is equal to 1 or 2,
   - q is equal to 0 or 1,
   - r is equal to 1 or 2,
   - R₄₂ and R₄₃, each represent, independently of each other, a (C₁-C₆)alkyl or an aryl group,
   - R₄₄ and R₄₅ each represent, independently of each other, a (C₁-C₆)alkyl or an aryl group, and
   - R₄₆, R₄₇ and R₆₈ each represent, independently of each other, a hydrogen atom, a (C₁-C₆)alkyl or an aryl group;
▪ **L_{X}** represents a bond, or a group selected from the group consisting of: 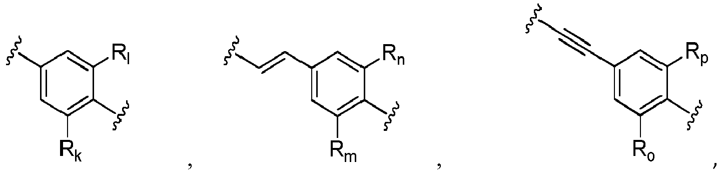 and 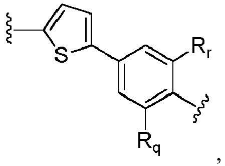
   wherein Rₖ, Rₗ, Rₘ, Rₙ, Rₒ, Rₚ, R_{q} and Rᵣ each represent, independently of each other, a halogen atom, a (C₁-C₆)alkyl, (C₁-C₆)haloalkyl, aryl, heteroaryl, aryl-(C₁-C₆)alkyl, heteroaryl-(C₁-C₆)alkyl, cycloalkyl, cycloalkyl-(C₁-C₆)alkyl, heterocycle, heterocycle-(C₁-C₆)alkyl, OR₄₈, SR₄₉, NR₅₀R₅₁, COR₅₂, CO₂R₅₃ or CONR₅₄R₅₅ group ; and
   **R₂** and **R₃** each represent, independently of each other, a hydrogen atom, a halogen atom, a (C₁-C₆)alkyl, (C₁-C₆)haloalkyl, (C₂-C₆)alkenyl, (C₂-C₆)alkynyl, (C₁-C₆)haloalkyl, aryl, heteroaryl, aryl-(C₁-C₆)alkyl, heteroaryl-(C₁-C₆)alkyl, cycloalkyl, cycloalkyl-(C₁-C₆)alkyl, heterocycle, heterocycle-(C₁-C₆)alkyl, OR₅₆, SR₅₇, NR₅₈R₅₉, COR₆₀, CO₂R₆₁ or CONR₆₂R₆₃ group ;
   with the proviso that:
   - when L_{X} represents a bond, R₂ and R₃ do not represent a hydrogen atom, and
   - when L_{X} does not represent a bond, R₂ and R₃ both represent a hydrogen atom; or
      Lx represents: 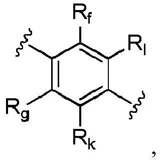 and
      R_{f} and R₂, and R_{g} and R₃ form together with the carbon atoms that carry them a cycloalkenyl or aryl group, wherein Rₖ and Rₗ are as defined above;
▪ R₅ to R₂₁, R₂₄, R₂₅, R₂₈ to R₄₁ and R₄₈ to R₆₃ each represent, independently of each other, a hydrogen atom, a (C₁-C₆)alkyl, (C₁-C₆)haloalkyl, aryl, heteroaryl, aryl-(C₁-C₆)alkyl, heteroaryl-(C₁-C₆)alkyl, cycloalkyl, cycloalkyl-(C₁-C₆)alkyl, heterocycle or heterocycle-(C₁-C₆)alkyl group, preferably a hydrogen atom or a (C₁-C₆)alkyl group, said group being optionally substituted by one or more groups selected from a halogen atom, a (C₁-C₆)alkyl, (C₁-C₆)haloalkyl, OR₆₄, SR₆₅ and NR₆₆R₆₇ group, wherein R₆₄ to R₆₇ each represent, independently of each other, a hydrogen atom or a (C₁-C₆)alkyl group; with the proviso that R₅, R₆, R₄₈, R₄₉, R₅₆ and R₅₇ are not H;
▪ R₂₂ to R₂₃ and R₂₆ to R₂₇ each represent, independently of each other a (C₁-C₂₀)alkyl, (C₁-C₂₀)haloalkyl, aryl, heteroaryl, aryl-(C₁-C₆)alkyl, heteroaryl-(C₁-C₆)alkyl, cycloalkyl, cycloalkyl-(C₁-C₆)alkyl, heterocycle or heterocycle-(C₁-C₆)alkyl group, preferably a (C₁-C₂₀)alkyl, notably (C₁-C₆)alkyl group, said group being optionally substituted by one or more groups selected from a halogen atom, a (C₁-C₆)alkyl, (C₁-C₆)haloalkyl, OR₆₄, SR₆₅ and NR₆₆R₆₇ group, wherein R₆₄ to R₆₇ each represent, independently of each other, a hydrogen atom or a (C₁-C₆)alkyl group;
   and
▪ **A⁺** represents a monovalent, organic or inorganic cation.

In particular, the present invention relates to a compound of the above formula (I), wherein:
▪ **X** represents an oxygen or a sulfur atom;
▪ **R₀** represents a hydrogen atom, a halogen atom, a (C₁-C₆)alkyl, (C₁-C₆)haloalkyl, aryl-(C₁-C₆)alkyl, heteroaryl-(C₁-C₆)alkyl, cycloalkyl, cycloalkyl-(C₁-C₆)alkyl, heterocycle, heterocycle-(C₁-C₆)alkyl, OR₅, SR₆, NR₇R₈, COR₁₁, CO₂R₁₂, CONR₁₃R₁₄, SO₂R₁₅, CN or NO₂ group, or is selected from the group consisting of: 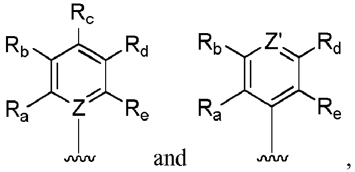 wherein:
   - Z represents C or N⁺A_{z}⁻ and Z' represents N or N⁺-R_{c}' A_{z}⁻, wherein
      A_{z}⁻ represents a monovalent organic or inorganic anion, and
      R_{c}' represents a (C₁-C₆)alkyl group,
   - Rₐ and Rₑ each represent, independently of each other, a hydrogen atom, a halogen atom, a (C₁-C₂₀)alkyl, (C₁-C₂₀)haloalkyl, aryl, heteroaryl, aryl-(C₁-C₆)alkyl, cycloalkyl, heterocycle, heterocycle-(C₁-C₆)alkyl, OR₂₂ or SR₂₃ group, and
   - R_{b}, R_{c} and R_{d} each represent, independently of each other, a hydrogen atom, a halogen atom or a (C₁-C₂₀)alkyl, (C₁-C₂₀)haloalkyl, cycloalkyl, heterocycle or O(C₁-C₂₀)alkyl group;
▪ **R₁** represents a halogen atom, a (C₁-C₂₀)alkyl, (C₂-C₆)alkynyl, (C₁-C₂₀)haloalkyl, aryl, heteroaryl, aryl-(C₁-C₆)alkyl, heteroaryl-(C₁-C₆)alkyl, cycloalkyl, cycloalkyl-(C₁-C₆)alkyl, heterocycle, heterocycle-(C₁-C₆)alkyl, (CH₂)ₘOR₂₄, (CH₂)_{m'}SR₂₅, OR₂₆, SR₂₇, NR₂₈R₂₉, COR₃₂, OCOR₃₆ or NR₃₈COR₃₉ group, wherein m and m' are, independently of each other, equal to 1, 2 or 3, preferably 1;
▪ **R₄** represents a halogen atom, a (C₁-C₂₀)alkyl, (C₂-C₆)alkynyl, (C₁-C₂₀)haloalkyl, aryl, heteroaryl, aryl-(C₁-C₆)alkyl, heteroaryl-(C₁-C₆)alkyl, cycloalkyl, cycloalkyl-(C₁-C₆)alkyl, heterocycle, heterocycle-(C₁-C₆)alkyl, (CH₂)ₘOR₂₄, (CH₂)_{m'}SR₂₅, OR₂₆, SR₂₇, NR₂₈R₂₉, COR₃₂, OCOR₃₆ or NR₃₈COR₃₉ group, wherein m and m' are, independently of each other, equal to 1, 2 or 3, preferably 1;
   or
   both R₄ groups form together a bond or a chain selected from the group consisting of
   - C(R₄₂R₄₃)-, -(CH₂)ₙ-, -Si(R₄₄R₄₅)-, -CH₂-Y-CH₂-, and -Y-CH₂-CH₂-Y'-, wherein:
      - Y and Y' each represent, independently of each other, O, S or NH,
      - n is equal to 2 or 3,
      - R₄₂ and R₄₃, each represent, independently of each other, a (C₁-C₆)alkyl or an aryl group, and
      - R₄₄ and R₄₅ each represent, independently of each other, a (C₁-C₆)alkyl or an aryl group;
▪ **L_{X}** represents a bond, or a group selected from the group consisting of: 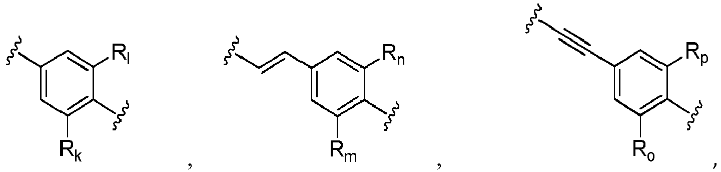 and 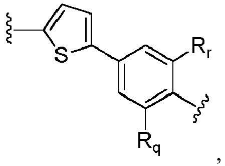
   wherein Rₖ, Rₗ, Rₘ, Rₙ, Rₒ, Rₚ, R_{q} and Rᵣ each represent, independently of each other, a halogen atom, a (C₁-C₆)alkyl, (C₁-C₆)haloalkyl, aryl, heteroaryl, cycloalkyl, heterocycle, OR₄₈, SR₄₉ or NR₅₀R₅₁ group ;
   and
   **R₂** and **R₃** each represent, independently of each other, a hydrogen atom, a halogen atom, a (C₁-C₆)alkyl, (C₁-C₆)haloalkyl, (C₂-C₆)alkenyl, (C₂-C₆)alkynyl, aryl, heteroaryl, cycloalkyl, heterocycle, OR₅₆, SR₅₇ or NR₅₈R₅₉ group ;
   with the proviso that:
      - when L_{X} represents a bond, R₂ and R₃ do not represent a hydrogen atom, and
      - when L_{X} does not represent a bond, R₂ and R₃ both represent a hydrogen atom; or
         **L_{X}** represents: 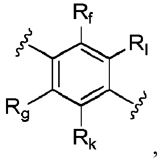 and R_{f} and R₂, and R_{g} and R₃ form together with the carbon atoms that carry them a cycloalkenyl or aryl group, wherein Rₖ and Rₗ are as defined above;
▪ R₅ to R₈, R₁₁ to R₁₅, R₂₄, R₂₅, R₂₈, R₂₉, R₃₂, R₃₆, R₃₈, R₃₉, R₄₈ to R₅₁, and R₅₆ to R₅₉ each represent, independently of each other, a hydrogen atom, a (C₁-C₆)alkyl, (C₁-C₆)haloalkyl, aryl, heteroaryl, aryl-(C₁-C₆)alkyl, heteroaryl-(C₁-C₆)alkyl, cycloalkyl, cycloalkyl-(C₁-C₆)alkyl, heterocycle or heterocycle-(C₁-C₆)alkyl group, preferably a hydrogen atom or a (C₁-C₆)alkyl group, said group being optionally substituted by one or more groups selected from a halogen atom, a (C₁-C₆)alkyl, (C₁-C₆)haloalkyl, OR₆₄, SR₆₅ and NR₆₆R₆₇ group, wherein R₆₄ to R₆₇ each represent, independently of each other, a hydrogen atom or a (C₁-C₆)alkyl group,
   with the proviso that R₅, R₆, R₄₈, R₄₉, R₅₆ and R₅₇ are not H;
▪ R₂₂ to R₂₃ and R₂₆ to R₂₇ each represent, independently of each other a (C₁-C₂₀)alkyl, (C₁-C₂₀)haloalkyl, aryl, heteroaryl, aryl-(C₁-C₆)alkyl, heteroaryl-(C₁-C₆)alkyl, cycloalkyl, cycloalkyl-(C₁-C₆)alkyl, heterocycle or heterocycle-(C₁-C₆)alkyl group, preferably a (C₁-C₂₀)alkyl, notably (C₁-C₆)alkyl group, said group being optionally substituted by one or more groups selected from a halogen atom, a (C₁-C₆)alkyl, (C₁-C₆)haloalkyl, OR₆₄, SR₆₅ and NR₆₆R₆₇ group, wherein R₆₄ to R₆₇ each represent, independently of each other, a hydrogen atom or a (C₁-C₆)-alkyl group;
   and
▪ A⁺ represents a monovalent, organic or inorganic cation.

As obvious to the skilled person, a compound of formula (I) exists in several mesomeric (or resonance) forms (or structures). The two main resonance structures of a compound of formula (I), which are represented below, are used indifferently throughout the present description:

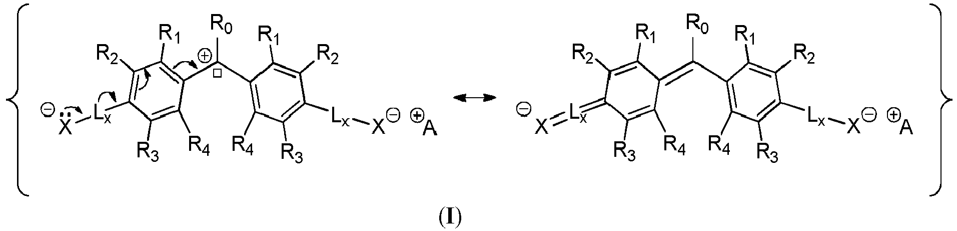

For the sake of clarity, the two main resonance structures of a compound of formula (I) depending on the nature of the L_{X} group are detailed in the following table 1:

**Table 1: main resonance structures of a compound of the invention**

| ***Lₓ*** | *Compound of formula* | *Main resonance structures* |
|---|---|---|
| Single bond | (**Ia**) | 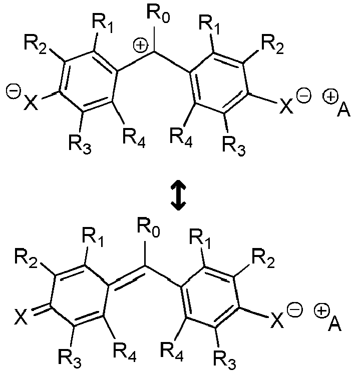 |
| 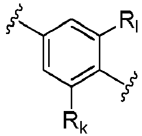 | (**Ib**) | 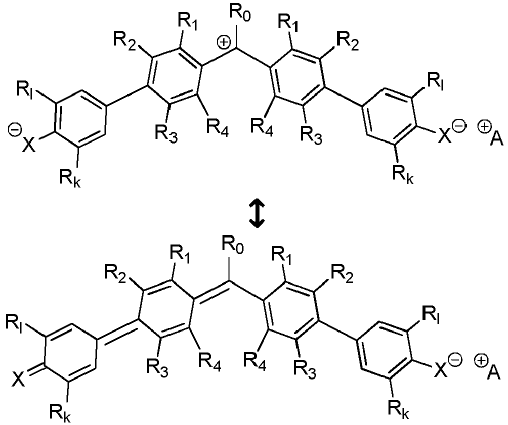 |
| 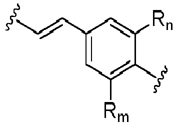 | **(Ic)** | 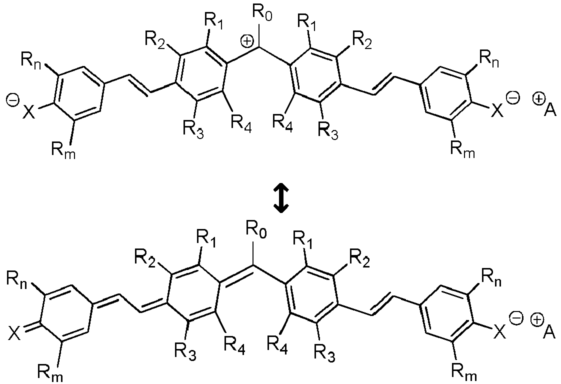 |
| 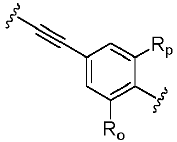 | (**Id**) | 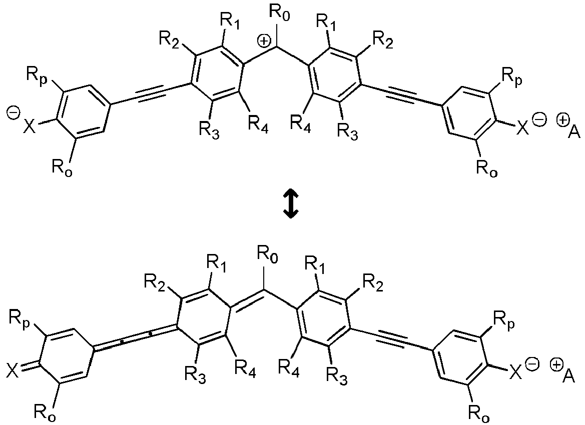 |
| 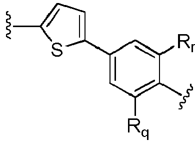 | (**Ie**) | 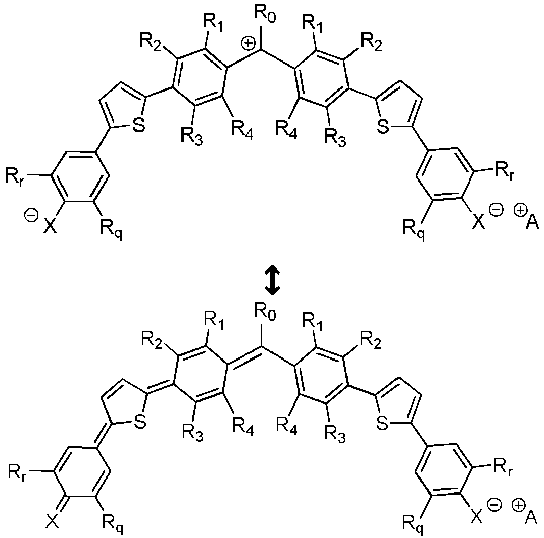 |

### DEFINITIONS

The term "halogen" as used in the present invention refers to an atom of fluorine, bromine, chlorine or iodine. Advantageously, this is an atom of bromine or fluorine.

The term "(C₁-C₆)alkyl" as used in the present invention refers to a saturated, linear or branched hydrocarbon chain comprising from 1 to 6 carbon atoms, including, but not limited to, methyl, ethyl, *n*-propyl, isopropyl, *n*-butyl, isobutyl, *sec*-butyl, *tert*-butyl, *n*-pentyl, *n*-hexyl and the like.

The term "(C₁-C₂₀)alkyl" as used in the present invention refers to a saturated, linear or branched hydrocarbon chain comprising from 1 to 20 carbon atoms, including, but not limited to, the (C₁-C₆)alkyl groups enumerated above, and octyl, decyl, dodecyl, tetradecyl, hexadecyl, octadecyl, eicosyl and the like.

The term "(C₁-C₆)haloalkyl" as used in the present invention refers to any (C₁-C₆)alkyl group as defined above in which one or more hydrogen atoms have been each replaced with a halogen atom. It can be notably a trifluoromethyl group.

The term "(C₁-C₂₀)haloalkyl" as used in the present invention refers to any (C₁-C₂₀)alkyl group as defined above in which one or more hydrogen atoms have been each replaced with a halogen atom. It can be notably perfluorooctyl, 1H,1H,2H,2H-perfluorooctyl, perfluorodecyl, 1H,1H,2H,2H-perfluorodecyl, perfluoroeicosyl and the like.

The term "(C₂-C₆)alkenyl" as used in the present invention refers to a linear or branched hydrocarbon chain comprising at least one double bond and comprising from 2 to 6 carbon atoms including, but not limited to, ethenyl (e.g. vinyl), propenyl (e.g. allyl) and the like.

The term "(C₂-C₆)alkynyl" as used in the present invention refers to a linear or branched hydrocarbon chain comprising at least one triple bond and comprising from 2 to 6 carbon atoms including, but not limited to, ethynyl, propynyl and the like.

The term "cycloalkyl" as used in the present invention refers to a saturated hydrocarbon ring comprising from 3 to 7, advantageously from 5 to 7, carbon atoms including, but not limited to, cyclohexyl, cyclopentyl, cyclopropyl, cycloheptyl and the like.

The term "cycloalkenyl" as used in the present invention refers to an unsaturated hydrocarbon ring comprising from 3 to 7, advantageously from 5 to 6, carbon atoms, including, but not limited to, cyclopentenyl, cyclohexenyl, cyclopentadienyl, cyclohexadienyl and the like.

The term "heterocycle" as used in the present invention refers to a saturated or unsaturated non-aromatic monocycle or polycycle, comprising fused, bridged or spiro rings, preferably fused rings, advantageously comprising 3 to 10, notably 3 to 6, atoms in each ring, in which the atoms of the ring(s) comprise one or more, advantageously 1 to 3, heteroatoms selected from O, S and N, preferably O and N, the remainder being carbon atoms.

A saturated heterocycle is more particularly a 3-, 4-, 5- or 6-membered, even more particularly a 5- or 6-membered saturated monocyclic heterocycle such as an aziridine, an azetidine, a pyrrolidine, a tetrahydrofuran, a 1,3-dioxolane, a tetrahydrothiophene, a thiazolidine, an isothiazolidine, an oxazolidine, an isoxazolidine, an imidazolidine, a pyrazolidine, a triazolidine, a piperidine, a piperazine, a 1,4-dioxane, a morpholine or a thiomorpholine.

An unsaturated heterocycle is more particularly an unsaturated monocyclic or bicyclic heterocycle, each cycle comprising 5 or 6 members, such as 1*H*-azirine, a pyrroline, a dihydrofuran, a 1,3-dioxolene, a dihydrothiophene, a thiazoline, an isothiazoline, an oxazoline, an isoxazoline, an imidazoline, a pyrazoline, a triazoline, a dihydropyridine, a tetrahydropyridine, a dihydropyrimidine, a tetrahydropyrimidine, a dihydropyridazine, a tetrahydropyridazine, a dihydropyrazine, a tetrahydropyrazine, a dihydrotriazine, a tetrahydrotriazine, a 1,4-dioxene, an indoline, a 2,3-dihydrobenzofuran (coumaran), a 2,3-dihydrobenzothiophene, a 1,3-benzodioxole, a 1,3-benzoxathiole, a benzoxazoline, a benzothiazoline, a benzimidazoline, a chromane or a chromene.

The term "aryl", as used in the present invention, refers to an aromatic hydrocarbon group comprising preferably 6 to 14 carbon atoms and comprising one or more fused rings, such as, for example, a phenyl, naphthyl or anthracenyl group. Advantageously, it will be a phenyl group.

The term "heteroaryl" as used in the present invention refers to an aromatic heterocycle as defined above. It can be more particularly an aromatic monocyclic, bicyclic or tricyclic heterocycle, each cycle comprising 5 or 6 members, such as a pyrrole, a furan, a thiophene, a thiazole, an isothiazole, an oxazole, an isoxazole, an imidazole, a pyrazole, a triazole, a pyridine, a pyrimidine, an indole, a benzofuran, a benzothiophene, a benzothiazole, a benzoxazole, a benzimidazole, an indazole, a benzotriazole, a quinoline, an isoquinoline, a cinnoline, a quinazoline, a quinoxaline, a carbazole, or a julolidine.

The term "cycloalkyl-(C₁-C₆)alkyl" as used in the present invention refers to any cycloalkyl group as defined above, which is bound to the molecule by means of a (C₁-C₆)-alkyl group as defined above.

The term "heterocycle-(C₁-C₆)alkyl" as used in the present invention refers to a heterocycle group as defined above, which is bound to the molecule by means of a (C₁-C₆)-alkyl group as defined above.

The term "aryl-(C₁-C₆)-alkyl" as used in the present invention refers to any aryl group as defined above, which is bound to the molecule by means of a (C₁-C₆)-alkyl group as defined above. In particular, it can be a benzyl group.

The term "heteroaryl-(C₁-C₆)alkyl" as used in the present invention refers to a heteroaryl group as defined above, which is bound to the molecule by means of a (C₁-C₆)-alkyl group as defined above.

The expression "organic or inorganic anion" refers, within the sense of the present invention, to a negatively-charged counter-ion. It can be in particular a halide (fluoride, chloride, bromide, iodide), perchlorate, nitrate, sulfate, alkylsulfate, benzenesulfonate, *p*-toluene sulfonate, chlorosulfonate, fluorosulfonate, trifluorosulfonate, methanesulfonate, benzenesulfinate, tetrafluoroborate, acetate, trifluoroacetate, propionate, benzoate, oxalate, succinate, oleate, stearate, citrate, hydrogenophosphate, dihydrogenophosphate or hexafluorophosphate anion. Preferably, it is a hexafluorophosphate, tetrafluoroborate, halide or triflate anion, more preferably a hexafluorophosphate anion.

The expression "organic or inorganic cation" refers, within the sense of the present invention, to a positively-charged counter-ion. It can be in particular an ammonium or a phosphonium ion or the conjugate acid of an organic nitrogen-containing bicyclic base.

Of note, within the context of the present invention, said inorganic cation might be present in a chelated form, the corresponding chelating agent being in particular a crown ether, preferably 18-crown-6.

The term "ammonium" as used in the present invention refers to any cation of the following formula N(R'R"R"'R"")⁺, wherein R', R", R'" and R"", each represent, independently of each other, an optionally substituted (C₁-C₂₀)alkyl, notably (C₁-C₆)alkyl, aryl or aryl-(C₁-C₆)-alkyl group, said group being as defined above. It can be in particular a tetrabutylammonium, a tetrapropylammonium, a tetraethylammonium, a tetramethylammonium, a benzyltrimethylammonium, a phenyltrimethylammonium, a hexadecylmethylammonium a triethylmethylammonium or a diethyldimethylammonium cation, or a choline.

The term "phosphonium" as used in the present invention refers to any cation of the following formula P(R'R"R"'R"")⁺, wherein R', R", R'" and R"" are as defined above. Preferably, R', R", R'" and R"", each represent, independently of each other, a (C₁-C₆)alkyl or aryl group. It can be notably a tetrabutylphosphonium or a tetraphenylphosphonium cation.

The term "organic nitrogen-containing bicyclic base", as used in the present invention, refers to a saturated or unsaturated, aromatic or non-aromatic bicycle, consisting in two fused, bridged or spiro rings, preferably fused or bridged rings, advantageously comprising 3 to 10, notably 4 to 8 atoms in each ring, in which the atoms of the ring(s) comprise one or more, advantageously 1 to 3, nitrogen atom(s), the remainder being carbon atoms. It can be notably 1,5-Diazabicyclo[4.3.0]non-5-ene (DBN), 1,8-Diazabicyclo[5.4.0]undec-7-ene (DBU), 1,5,7-Triazabicyclo[4.4.0]dec-5-ene (TBD) or 1,4-Diazabicyclo[2.2.2]octane (DABCO).

In the context of the present invention, "C⁺" refers to the trivalent carbon atom that is linked to R₀.

In the context of the present invention, the terms "precursor" or "intermediate" are used indifferently.

### DETAILED DESCRIPTION AND ADDITIONAL EMBODIMENTS

In a preferred embodiment, **X** represents an oxygen atom.

According to a particular embodiment, **R₀** represents a hydrogen atom, a halogen atom, a (C₁-C₆)alkyl, (C₁-C₆)haloalkyl, aryl-(C₁-C₆)alkyl, heteroaryl-(C₁-C₆)alkyl, cycloalkyl, cycloalkyl-(C₁-C₆)alkyl, heterocycle, heterocycle-(C₁-C₆)alkyl, OR₅, SR₆, NR₇R₈, CN or NO₂ group, or is selected from the group consisting of: 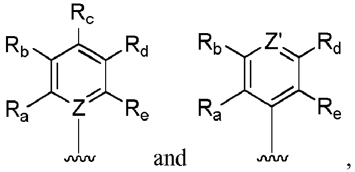 wherein:
- Z represents C or N⁺A_{z}⁻ and Z' represents N or N⁺-R_{c}' A_{z}⁻, wherein
   A_{z}⁻ represents a monovalent organic or inorganic anion, and
   R_{c}' represents a (C₁-C₆)alkyl group,
- Rₐ and Rₑ each represent, independently of each other, a hydrogen atom, a halogen atom, a (C₁-C₂₀)alkyl, (C₁-C₂₀)haloalkyl, aryl, heteroaryl, cycloalkyl, heterocycle, OR₂₂ or SR₂₃ group, and
- R_{b}, R_{c} and R_{d} each represent, independently of each other, a hydrogen atom, a halogen atom or a (C₁-C₂₀)alkyl, (C₁-C₂₀)haloalkyl, cycloalkyl, heterocycle or O(C₁-C₂₀)alkyl group.

According to another particular embodiment, **R₀** represents a hydrogen atom, a halogen atom, a (C₁-C₆)alkyl, (C₁-C₆)haloalkyl, such as CF₃, cycloalkyl, heterocycle, OR₅, SR₆, NR₇R₈, CN or NO₂ group, or is selected from the group consisting of: 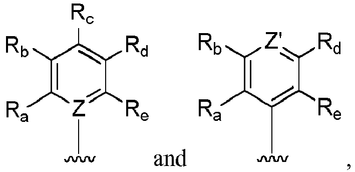 wherein:
- Z represents C or N⁺A_{z}⁻ and Z' represents N or N⁺-R_{c}' A_{z}⁻, wherein
   A_{z}⁻ represents a monovalent organic or inorganic anion, and
   R_{c}' represents a (C₁-C₆)alkyl group,
- Rₐ and Rₑ each represent, independently of each other a hydrogen atom, a halogen atom, a (C₁-C₆)alkyl, (C₁-C₆)haloalkyl, OR₂₂ or SR₂₃ group, and
- R_{b}, R_{c} and R_{d} each represent, independently of each other, a hydrogen atom, a halogen atom or a O(C₁-C₂₀)alkyl, (C₁-C₆)alkyl or (C₁-C₆)haloalkyl group.

According to still another particular embodiment, **R₀** represents a hydrogen atom, a halogen atom, a (C₁-C₆)alkyl, (C₁-C₆)haloalkyl, such as CF₃, cycloalkyl, heterocycle, OR₅, SR₆, NR₇R₈, CN or NO₂ group, or is: 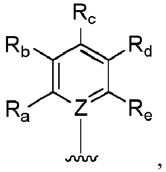 wherein:
- Z represents C,
- Rₐ and Rₑ each represent, independently of each other a hydrogen atom, a halogen atom, a (C₁-C₆)alkyl, (C₁-C₆)haloalkyl, OR₂₂ or SR₂₃ group, and
- R_{b}, R_{c} and R_{d} each represent, independently of each other, a hydrogen atom, a halogen atom or a O(C₁-C₂₀)alkyl, (C₁-C₆)alkyl or (C₁-C₆)haloalkyl group.

According to yet another particular embodiment, **R₀** represents a hydrogen atom, a halogen atom, a (C₁-C₆)alkyl, (C₁-C₆)haloalkyl, such as CF₃, OR₅, NR₇R₈, CN or NO₂ group, or is: 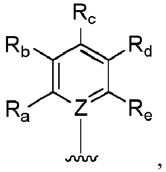 wherein:
- Z represents C,
- Rₐ and Rₑ each represent, independently of each other, a hydrogen atom, a halogen atom, a (C₁-C₆)alkyl, (C₁-C₆)haloalkyl or OR₂₂ group, and
- R_{b}, R_{c} and R_{d} each represent a O(C₁-C₂₀)alkyl group or a hydrogen atom.

In the above embodiments, Rₐ and Rₑ are preferably the same.

In the above embodiments, R_{b}, R_{c} and R_{d} are preferably the same.

In a preferred embodiment, R₀ represents a hydrogen atom, a (C₁-C₆)alkyl, OR₅, NR₇R₈, or a CN group, notably a hydrogen atom, NR₇R₈, or a CN group, advantageously a hydrogen atom.

According to a particular embodiment, **R₁** represents a halogen atom, a (C₁-C₂₀)alkyl, notably (C₁-C₆)alkyl, (C₁-C₂₀)haloalkyl, notably (C₁-C₆)haloalkyl, aryl, heteroaryl, cycloalkyl, heterocycle, (CH₂)ₘOR₂₄, (CH₂)_{m'}SR₂₅, OR₂₆, SR₂₇ or NR₂₈R₂₉ group, wherein m and m' are, independently of each other, equal to 1, 2 or 3, preferably 1.

According to another particular embodiment, **R₁** represents a halogen atom, a (C₁-C₆)alkyl, (C₁-C₆)haloalkyl, aryl, (CH₂)ₘOR₂₄, (CH₂)_{m'}SR₂₅, OR₂₆, SR₂₇ or NR₂₈R₂₉, group, wherein m and m' are, independently of each other, equal to 1, 2 or 3, preferably 1.

According to yet another embodiment, **R₁** represents a halogen atom, a (C₁-C₆)alkyl, (C₁-C₆)haloalkyl, aryl, CH₂-OR₂₄ or OR₂₆ group, preferably a (C₁-C₆)alkyl, aryl, CH₂-OR₂₄ or OR₂₆ group, notably a (C₁-C₆)alkyl group.

According to a particular embodiment, **R₄** represents a halogen atom, a (C₁-C₂₀)alkyl, notably (C₁-C₆)alkyl, (C₁-C₂₀)haloalkyl, notably (C₁-C₆)haloalkyl, aryl, heteroaryl, cycloalkyl, heterocycle, (CH₂)ₘOR₂₄, (CH₂)_{m'}SR₂₅, OR₂₆, SR₂₇ or NR₂₈R₂₉ group, wherein m and m' are, independently of each other, equal to 1, 2 or 3, preferably 1;
or
both R₄ groups form together a bond or a chain selected from the group consisting of -C(R₄₂R₄₃)-, -(CH₂)ₙ-, -Si(R₄₄R₄₅)- and -Y-CH₂-CH₂-Y'-, wherein:
- Y and Y' each represent, independently of each other, O, S or NH,
- n is equal to 2 or 3,
- R₄₂ and R₄₃, each represent, independently of each other, a (C₁-C₆)alkyl or an aryl group, preferably a (C₁-C₆)alkyl group, and
- R₄₄ and R₄₅ each represent, independently of each other, a (C₁-C₆)alkyl or an aryl group, preferably a (C₁-C₆)alkyl group.

According to another particular embodiment, **R₄** represents a halogen atom, a (C₁-C₆)alkyl, (C₁-C₆)haloalkyl, aryl, (CH₂)ₘOR₂₄, (CH₂)_{m'}SR₂₅, OR₂₆, SR₂₇ or NR₂₈R₂₉, group, wherein m and m' are, independently of each other, equal to 1, 2 or 3, preferably 1;
or
both R₄ groups form together a bond or a chain selected from the group consisting of-C(R₄₂R₄₃)-, -(CH₂)ₙ-, -Si(R₄₄R₄₅)- and -Y-CH₂-CH₂-Y'-, wherein:
- Y and Y' each represent, independently of each other, O, S or NH,
- n is equal to 2 or 3,
- R₄₂ and R₄₃, each represent, independently of each other, a (C₁-C₆)alkyl group, and
- R₄₄ and R₄₅ each represent, independently of each other, a (C₁-C₆)alkyl group.

According to yet another embodiment, **R₄** represents a halogen atom, a (C₁-C₆)alkyl, (C₁-C₆)haloalkyl, aryl, CH₂-OR₂₄ or OR₂₆ group,
or
both R₄ groups form together a bond or a chain selected from the group consisting of-C(R₄₂R₄₃)-, -CH₂-CH₂-, -Si(R₄₄R₄₅)- and -O-CH₂-CH₂-O-, wherein R₄₂ to R₄₅, each represent, independently of each other, a (C₁-C₆)alkyl group.

According to a particular embodiment, **R₄** represents a halogen atom, a (C₁-C₂₀)alkyl, notably (C₁-C₆)alkyl, (C₁-C₂₀)haloalkyl, notably (C₁-C₆)haloalkyl, aryl, heteroaryl, cycloalkyl, heterocycle, (CH₂)ₘOR₂₄, (CH₂)_{m'}SR₂₅, OR₂₆, SR₂₇ or NR₂₈R₂₉ group, wherein m and m' are, independently of each other, equal to 1, 2 or 3, preferably 1.

According to another particular embodiment, **R₄** represents a halogen atom, a (C₁-C₆)alkyl, (C₁-C₆)haloalkyl, aryl, (CH₂)ₘOR₂₄, (CH₂)_{m'}SR₂₅, OR₂₆, SR₂₇ or NR₂₈R₂₉, group, wherein m and m' are, independently of each other, equal to 1, 2 or 3, preferably 1.

According to yet another embodiment, **R₄** represents a halogen atom, a (C₁-C₆)alkyl, (C₁-C₆)haloalkyl, aryl, CH₂-OR₂₄ or OR₂₆ group, preferably a (C₁-C₆)alkyl, aryl, CH₂-OR₂₄ or OR₂₆ group, notably a (C₁-C₆)alkyl group.

In a preferred embodiment, **R₁** and **R₄** are the same, and are as defined in any of the above embodiments.

According to a particular embodiment, **L_{X}** represents a bond, or a group selected from the group consisting of: 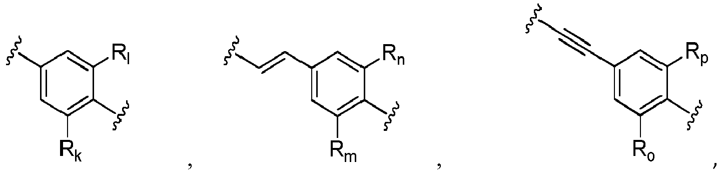 and 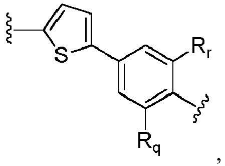
wherein Rₖ, Rₗ, Rₘ, Rₙ, Rₒ, Rₚ, R_{q} and Rᵣ each represent, independently of each other, a halogen atom, a (C₁-C₆)alkyl, (C₁-C₆)haloalkyl, aryl, heteroaryl, cycloalkyl, heterocycle, OR₄₈, SR₄₉ or NR₅₀R₅₁ group ;
and
**R₂** and **R₃** each represent, independently of each other, a hydrogen atom, a halogen atom, a (C₁-C₆)alkyl, (C₁-C₆)haloalkyl, aryl, heteroaryl, cycloalkyl, heterocycle, OR₅₆, SR₅₇ or NR₅₈R₅₉ group ;
with the proviso that:
   - when L_{X} represents a bond, R₂ and R₃ do not represent a hydrogen atom, and
   - when L_{X} does not represent a bond, R₂ and R₃ both represent a hydrogen atom; or L_{X} represents: 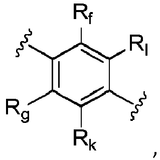
   and
R_{f} and R₂, and R_{g} and R₃ form together with the carbon atoms that carry them a cycloalkenyl or aryl group, wherein Rₖ and Rₗ are as defined above.

According to another particular embodiment, **L_{X}** represents a bond, or a group selected from the group consisting of: 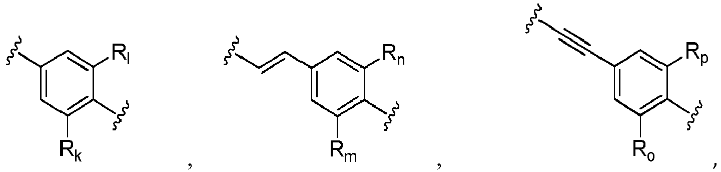 and 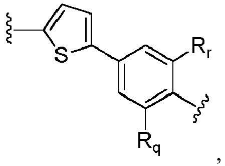
wherein Rₖ, Rₗ, Rₘ, Rₙ, Rₒ, Rₚ, R_{q} and Rᵣ each represent, independently of each other, a halogen atom, a (C₁-C₆)alkyl, (C₁-C₆)haloalkyl, aryl, heteroaryl, cycloalkyl, heterocycle, OR₄₈, SR₄₉ or NR₅₀R₅₁ group ;
and
**R₂** and **R₃** each represent, independently of each other, a hydrogen atom, a halogen atom, a (C₁-C₆)alkyl, (C₁-C₆)haloalkyl, aryl, heteroaryl, cycloalkyl, heterocycle, OR₅₆, SR₅₇ or NR₅₈R₅₉ group ; with the proviso that:
   - when L_{X} represents a bond, R₂ and R₃ do not represent a hydrogen atom, and
   - when L_{X} does not represent a bond, R₂ and R₃ both represent a hydrogen atom.

According to still another particular embodiment, **L_{X}** represents a bond, or a group selected from the group consisting of: 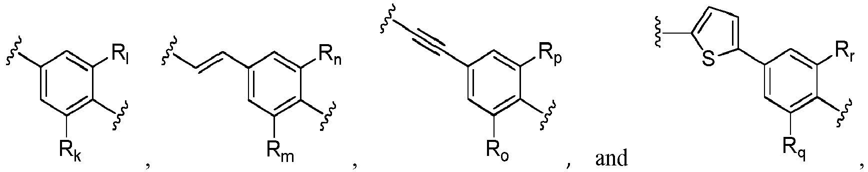
wherein Rₖ, Rₗ, Rₘ, Rₙ, Rₒ, Rₚ, R_{q} and Rᵣ each represent, independently of each other, a halogen atom, a (C₁-C₆)alkyl, (C₁-C₆)haloalkyl, such as CF₃, aryl, heteroaryl, cycloalkyl or heterocycle group ;
and
**R₂** and **R₃** each represent, independently of each other, a hydrogen atom, a halogen atom, a (C₁-C₆)alkyl, (C₁-C₆)haloalkyl, such as CF₃, aryl, heteroaryl, cycloalkyl, heterocycle, OR₅₆, SR₅₇, or NR₅₈R₅₉ group ; with the proviso that:
   - when L_{X} represents a bond, R₂ and R₃ do not represent a hydrogen atom, and
   - when L_{X} does not represent a bond, R₂ and R₃ both represent a hydrogen atom.

According to yet another particular embodiment, **L_{X}** represents a bond, or a group selected from the group consisting of: 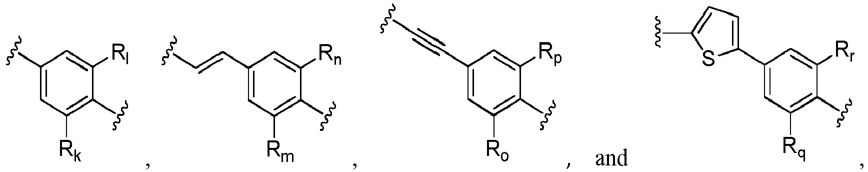
wherein Rₖ, Rₗ, Rₘ, Rₙ, Rₒ, Rₚ, R_{q} and Rᵣ each represent, independently of each other, a halogen atom, a (C₁-C₆)alkyl, (C₁-C₆)haloalkyl, such as CF₃ or aryl group ;
and
**R₂** and **R₃** each represent, independently of each other, a hydrogen atom, a halogen atom, a (C₁-C₆)alkyl, (C₁-C₆)haloalkyl, such as CF₃, aryl, OR₅₆, SR₅₇, or NR₅₈R₅₉ group ; with the proviso that:
   - when L_{X} represents a bond, R₂ and R₃ do not represent a hydrogen atom, and
   - when L_{X} does not represent a bond, R₂ and R₃ both represent a hydrogen atom.

Preferably, in the above embodiments, **R₂** and **R₃** are the same.

Preferably, in the above embodiments, Rₖ and Rₗ are the same, Rₘ and Rₙ are the same, Rₒ and Rₚ are the same, and R_{q} and Rᵣ are the same.

In another particular embodiment, **L_{X}** represents a bond and **R₂** and **R₃** each represent, independently of each other, a halogen atom, a (C₁-C₆)alkyl, (C₁-C₆)haloalkyl, such as CF₃, aryl, heteroaryl, cycloalkyl, heterocycle, OR₅₆, SR₅₇ or NR₅₈R₅₉ group;
or **L_{X}** represents 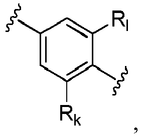 wherein Rₖ and Rₗ each represent, independently of each other, a halogen atom, a (C₁-C₆)alkyl, (C₁-C₆)haloalkyl, such as CF₃, aryl, heteroaryl, cycloalkyl or heterocycle group, preferably a halogen atom, a (C₁-C₆)alkyl, (C₁-C₆)haloalkyl, aryl, heteroaryl, cycloalkyl, heterocycle, OR₄₈, SR₄₉ or NR₅₀R₅₁ group, and **R₂** and **R₃** both represent a hydrogen atom.

In still another particular embodiment, **L_{X}** represents a bond and **R₂** and **R₃** each represent, independently of each other, a halogen atom, a (C₁-C₆)alkyl, (C₁-C₆)haloalkyl, such as CF₃, aryl, heteroaryl, OR₅₆, SR₅₇ or NR₅₈R₅₉group;
or **L_{X}** represents 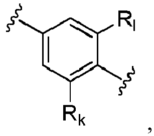 wherein Rₖ and Rₗ, each represent, independently of each other, a halogen atom, a (C₁-C₆)alkyl, (C₁-C₆)haloalkyl, an aryl or a heteroaryl group, and **R₂** and **R₃** both represent a hydrogen atom.

In yet another particular embodiment, **L_{X}** represents a bond and **R₂** and **R₃** each represent, independently of each other, a halogen atom, a (C₁-C₆)alkyl, aryl or OR₅₆ group ;
or **L_{X}** represents 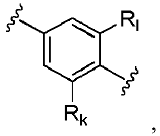 wherein Rₖ and Rₗ, each represent, independently of each other, a halogen atom, a (C₁-C₆)alkyl or aryl group, and **R₂** and **R₃** both represent a hydrogen atom.

Preferably, in the above embodiments, **R₂** and **R₃** are the same, and Rₖ and Rₗ are the same.

In a preferred embodiment, **L_{X}** represents a bond and **R₂** and **R₃** each represent, independently of each other, a halogen atom, a (C₁-C₆)alkyl, (C₁-C₆)haloalkyl, aryl, heteroaryl, cycloalkyl, heterocycle, OR₅₆, SR₅₇ or NR₅₈R₅₉ group, notably a halogen atom, a (C₁-C₆)alkyl, (C₁-C₆)haloalkyl, such as CF₃, aryl, heteroaryl, OR₅₆, SR₅₇ or NR₅₈R₅₉ group, in particular a halogen atom, a (C₁-C₆)alkyl, aryl or OR₅₆ group. Preferably, **R₂** and **R₃** are the same.

In all of the above embodiments:
R₅ to R₈, R₁₁ to R₁₅, R₂₄, R₂₅, R₂₈, R₂₉, R₃₂, R₃₆, R₃₈, R₃₉, R₄₈ to R₅₁, and R₅₆ to R₅₉ each represent, independently of each other, a hydrogen atom, a (C₁-C₆)alkyl, (C₁-C₆)haloalkyl, aryl, heteroaryl, aryl-(C₁-C₆)alkyl, heteroaryl-(C₁-C₆)alkyl, cycloalkyl, cycloalkyl-(C₁-C₆)alkyl, heterocycle or heterocycle-(C₁-C₆)alkyl group, preferably a hydrogen atom or a (C₁-C₆)alkyl group, said group being optionally substituted by one or more groups selected from a halogen atom, a (C₁-C₆)alkyl, (C₁-C₆)haloalkyl, OR₆₄, SR₆₅ and NR₆₆R₆₇ group, wherein R₆₄ to R₆₇ each represent, independently of each other, a hydrogen atom or a (C₁-C₆)alkyl group,
   with the proviso that R₅, R₆, R₄₈, R₄₉, R₅₆ and R₅₇ are not H;
in particular, R₅ to R₈, R₁₁ to R₁₅, R₂₄, R₂₅, R₂₈, R₂₉, R₃₂, R₃₆, R₃₈, R₃₉, R₄₈ to R₅₁, and R₅₆ to R₅₉ each represent, independently of each other, a (C₁-C₆)alkyl group;
and
R₂₂ to R₂₃ and R₂₆ to R₂₇ each represent, independently of each other a (C₁-C₂₀)alkyl, notably (C₁-C₆)alkyl, (C₁-C₂₀)haloalkyl, notably (C₁-C₂₀)haloalkyl, aryl, heteroaryl, aryl-(C₁-C₆)alkyl, heteroaryl-(C₁-C₆)alkyl, cycloalkyl, cycloalkyl-(C₁-C₆)alkyl, heterocycle or heterocycle-(Ci-C₆)alkyl group, preferably a (C₁-C₂₀)alkyl, notably (C₁-C₆)alkyl group, said group being optionally substituted by one or more groups selected from a halogen atom, a (C₁-C₆)alkyl, (C₁-C₆)haloalkyl, OR₆₄, SR₆₅ and NR₆₆R₆₇ group, wherein R₆₄ to R₆₇ each represent, independently of each other, a hydrogen atom or a (C₁-C₆)-alkyl group;
in particular, R₂₂ to R₂₃ and R₂₆ to R₂₇ each represent, independently of each other a (C₁-C₂₀)alkyl, notably (C₁-C₆)alkyl group.

In particular, the invention relates to a compound of formula (**I**), wherein:
▪ **X** represents an oxygen or a sulfur atom;
▪ **R₀** represents a hydrogen atom, a halogen atom, a (C₁-C₆)alkyl, (C₁-C₆)haloalkyl, such as CF₃, cycloalkyl, heterocycle, OR₅, SR₆, NR₇R₈, CN or NO₂ group, or is: 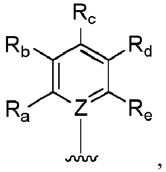 wherein:
   - Z represents C,
   - Rₐ and Rₑ are the same and represent a hydrogen atom, a halogen atom, a (C₁-C₂₀)alkyl, notably (C₁-C₆)alkyl, (C₁-C₂₀)haloalkyl, notably (C₁-C₆)haloalkyl, OR₂₂ or SR₂₃ group, and
   - R_{b}, R_{c} and R_{d} each represent, independently of each other, a hydrogen atom, a halogen atom or a (C₁-C₆)alkyl or a O(C₁-C₂₀)alkyl group, preferably R_{b}, R_{c} and R_{d} are the same;
▪ **R₁** and **R₄** are the same, and represent a halogen atom, a (C₁-C₂₀)alkyl, notably (C₁-C₆)alkyl, (C₁-C₂₀)haloalkyl, notably (C₁-C₆)haloalkyl, aryl, heteroaryl, cycloalkyl, heterocycle, (CH₂)ₘOR₂₄, (CH₂)_{m'}SR₂₅, OR₂₆, SR₂₇ or NR₂₈R₂₉ group, wherein m and m' are, independently of each other, equal to 1, 2 or 3, preferably 1; and
▪ **L_{X}** represents a bond, or a group selected from the group consisting of: 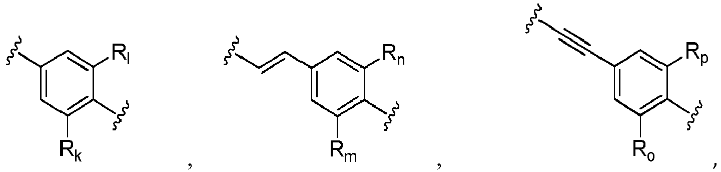 and 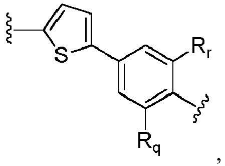
   wherein Rₖ and Rₗ are the same, Rₘ and Rₙ are the same, Rₒ and Rₚ are the same, R_{q} and Rᵣ are the same, and represent a halogen atom, a (C₁-C₆)alkyl, (C₁-C₆)haloalkyl, aryl, heteroaryl, cycloalkyl, heterocycle, OR₄₈, SR₄₉ or NR₅₀R₅₁ group ;
   and
   **R₂** and **R₃** are the same and represent a hydrogen atom, a halogen atom, a (C₁-C₆)alkyl, (C₁-C₆)haloalkyl, aryl, heteroaryl, cycloalkyl, heterocycle, OR₅₆, SR₅₇ or NR₅₈R₅₉ group ; with the proviso that:
      - when L_{X} represents a bond, R₂ and R₃ do not represent a hydrogen atom, and
      - when L_{X} does not represent a bond, R₂ and R₃ both represent a hydrogen atom.

Notably, the invention relates to a compound of formula (**I**), wherein:
▪ **X** represents an oxygen atom;
▪ **R₀** represents a hydrogen atom, a halogen atom, a (C₁-C₆)alkyl, (C₁-C₆)haloalkyl, such as CF₃, OR₅, NR₇R₈, CN or NO₂ group, or is: 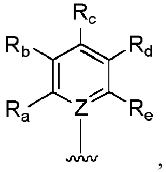 wherein:
   - Z represents C,
   - Rₐ and Rₑ are the same and represent, independently of each other, a hydrogen atom, a halogen atom, a (C₁-C₆)alkyl, (C₁-C₆)haloalkyl or OR₂₂ group, and
   - R_{b}, R_{c} and R_{d} each represent a hydrogen atom or a O(C₁-C₂₀)alkyl group, preferably R_{b}, R_{c} and R_{d} are the same;
▪ **R₁** and **R₄** are the same, and represent a halogen atom, a (C₁-C₆)alkyl, (C₁-C₆)haloalkyl, aryl, (CH₂)ₘOR₂₄, (CH₂)_{m'}SR₂₅, OR₂₆, SR₂₇ or NR₂₈R₂₉, group, wherein m and m' are, independently of each other, equal to 1, 2 or 3, preferably 1; and
▪ **L_{X}** represents a bond and **R₂** and **R₃** are the same and represent a halogen atom, a (C₁-C₆)alkyl, (C₁-C₆)haloalkyl, such as CF₃, aryl, heteroaryl, cycloalkyl, heterocycle, OR₅₆, SR₅₇ or NR₅₈R₅₉ group;
   or **L_{X}** represents 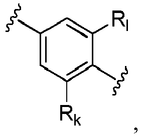
wherein Rₖ and Rₗ are the same and represent a halogen atom, a (C₁-C₆)alkyl, (C₁-C₆)haloalkyl, such as CF₃, aryl, heteroaryl, cycloalkyl or heterocycle group, preferably a halogen atom, a (C₁-C₆)alkyl, (C₁-C₆)haloalkyl, aryl, heteroaryl, cycloalkyl, heterocycle, OR₄₈, SR₄₉ or NR₅₀R₅₁ group, and **R₂** and **R₃** both represent a hydrogen atom.

In a particular embodiment, the invention relates to a compound of formula (I), wherein:
▪ **X** represents an oxygen atom;
▪ **R₀** represents a hydrogen atom, a (C₁-C₆)alkyl, OR₅, NR₇R₈ or a CN group, notably a hydrogen atom, NR₇R₈ or a CN group, advantageously a hydrogen atom;
▪ **R₁** and **R₄** are the same, and represent a (C₁-C₆)alkyl, (C₁-C₆)haloalkyl, aryl, CH₂-OR₂₄ or OR₂₆ group, preferably a (C₁-C₆)alkyl, CH₂-OR₂₄ or OR₂₆ group, notably a (C₁-C₆)alkyl group;
▪ Lx represents a bond; and
▪ **R₂** and **R₃** are the same and represent a halogen atom, a (C₁-C₆)alkyl, (C₁-C₆)haloalkyl, such as CF₃, aryl, heteroaryl, OR₅₆, SR₅₇ or NR₅₈R₅₉ group, in particular a halogen atom, a (C₁-C₆)alkyl, aryl or OR₅₆ group.

In all of the above embodiments, R₅ to R₈, R₁₁ to R₁₅, R₂₂ to R₂₉, R₃₂, R₃₆, R₃₈, R₃₉, R₄₈ to R₅₁, and R₅₆ to R₅₉ are as defined above. In particular, R₅ to R₈, R₁₁ to R₁₅, R₂₄, R₂₅, R₂₈, R₂₉, R₃₂, R₃₆, R₃₈, R₃₉, R₄₈ to R₅₁, and R₅₆ to R₅₉ each represent, independently of each other, a (C₁-C₆)alkyl group; and R₂₂ to R₂₃ and R₂₆ to R₂₇ each represent, independently of each other a (C₁-C₂₀)alkyl, notably (C₁-C₆)alkyl group.

In another particular embodiment, a compound of the present invention is chosen among the following compounds:

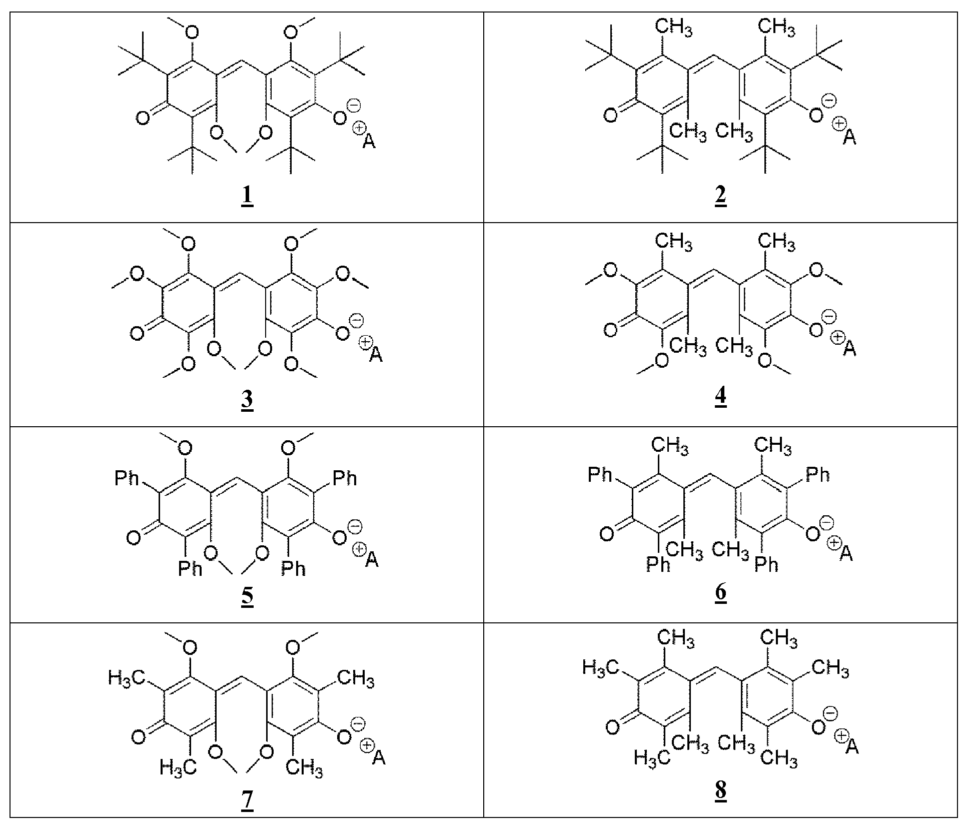

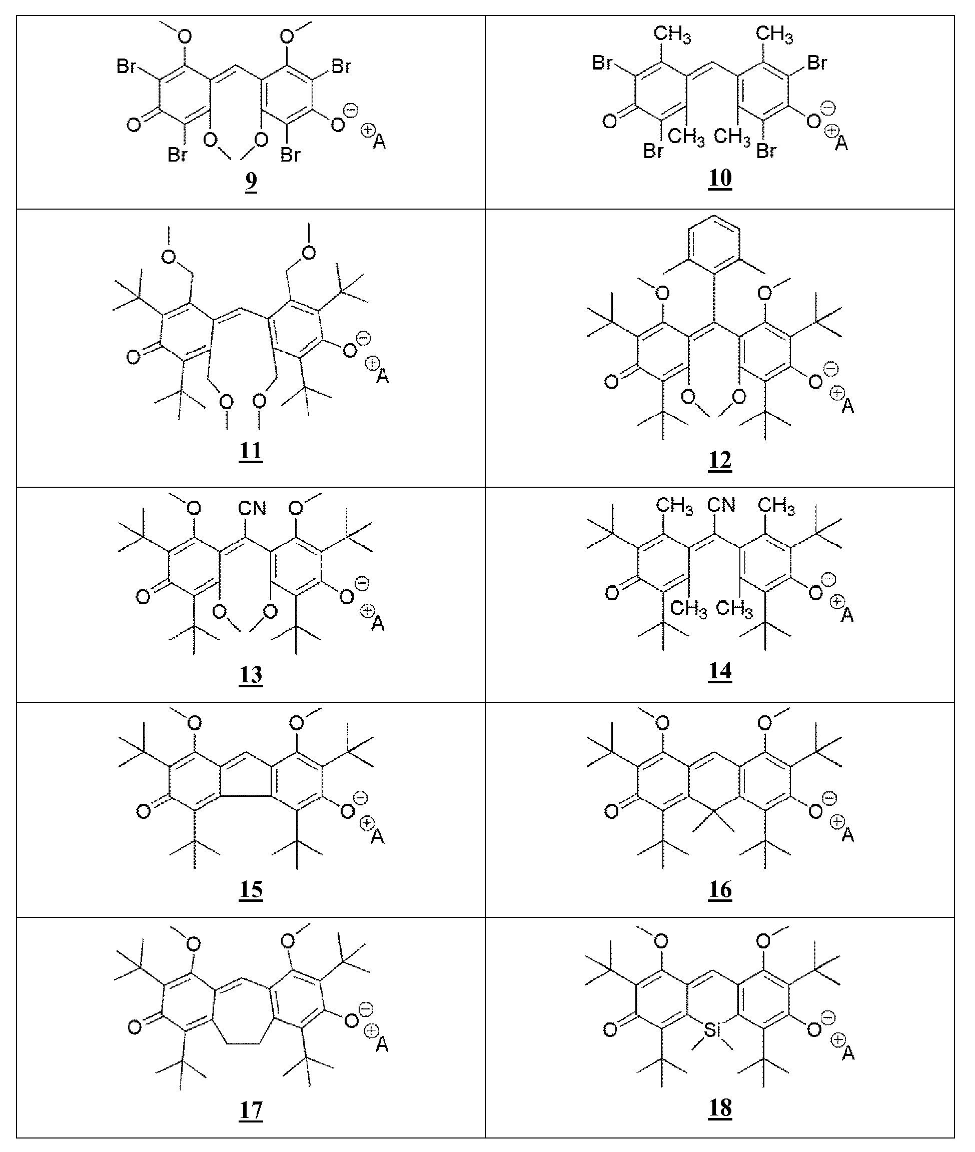

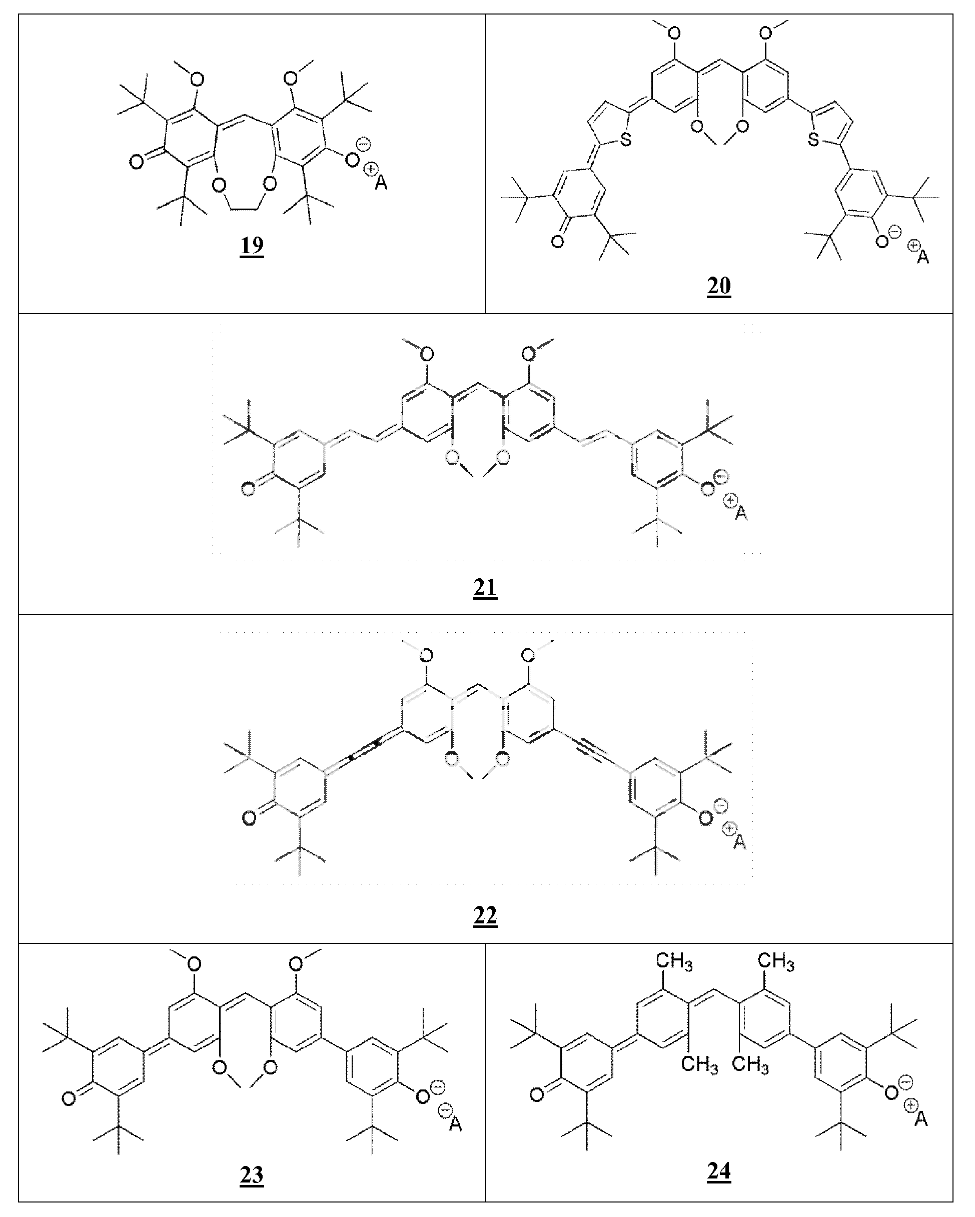

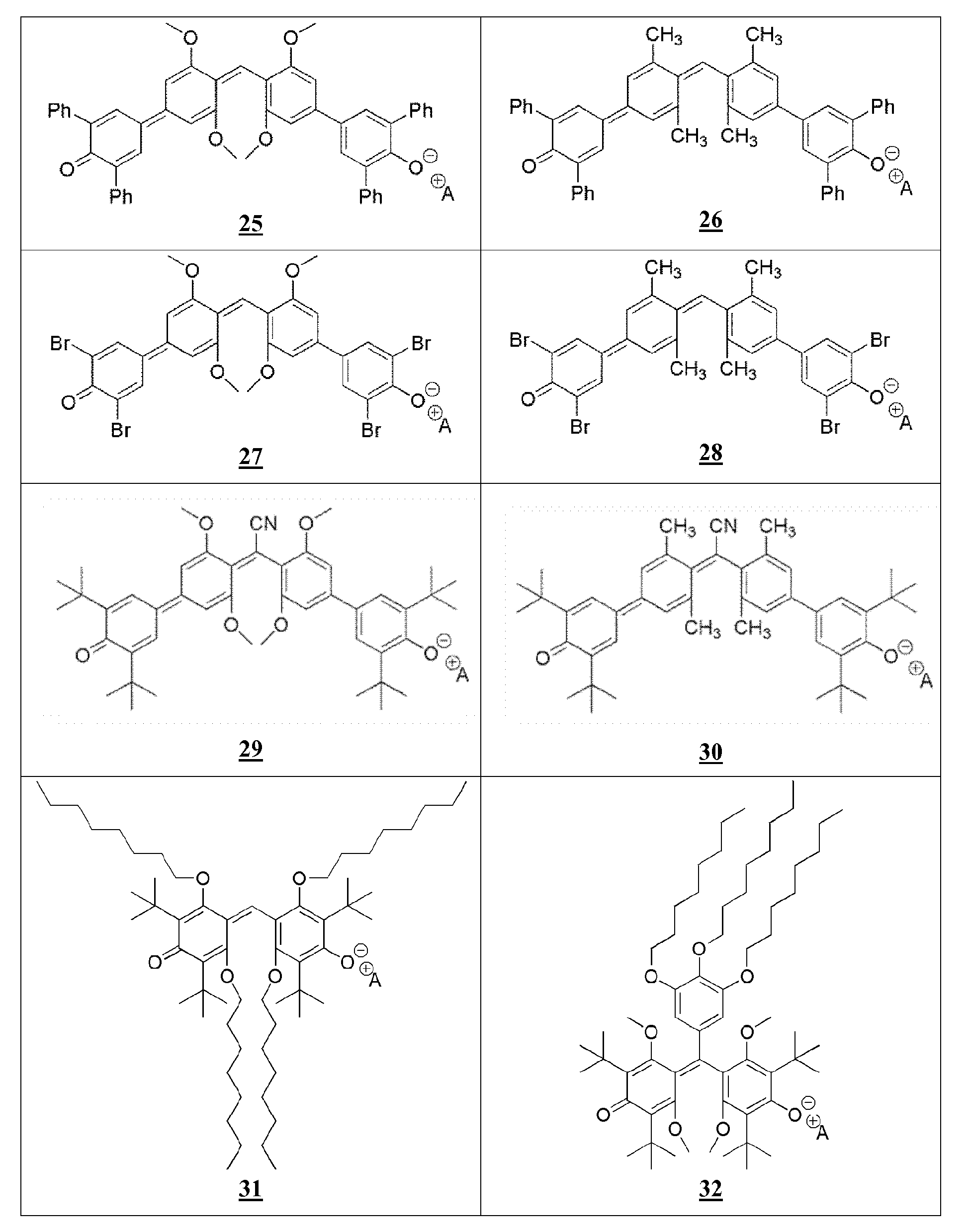

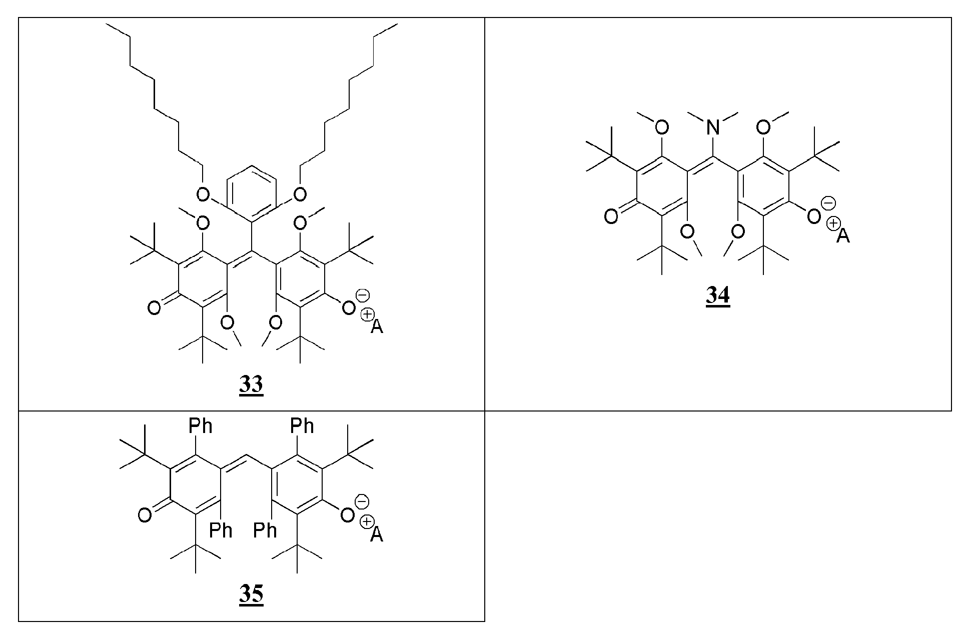

Notably, a compound of the present invention is chosen among compounds **1 to 6, 8** to **10, 13, 23, 24, 32** and **34,** in particular among compounds **1, 2** and **8.** Advantageously, a compound of the present invention is compound **8.**

In all of the above embodiments and compounds, **A**⁺ represents a monovalent, organic or inorganic cation.

In particular, **A**⁺ represents an ammonium or a phosphonium ion or the conjugate acid of an organic nitrogen-containing bicyclic base.

Advantageously, **A**⁺ is selected from the group consisting of tetrabutylammonium, tetrapropylammonium, tetraethylammonium, tetramethylammonium, benzyltrimethylammonium, phenyltrimethylammonium, hexadecylmethylammonium choline, triethylmethylammonium, diethyldimethylammonium, tetrabutylphosphonium, tetraphenylphosphonium, DBN-H⁺, DBU-H⁺, TBD-H⁺ and DABCO-H⁺, preferably from the group consisting of tetrabutylammonium, tetrapropylammonium, tetraethylammonium, tetramethylammonium, benzyltrimethylammonium, phenyltrimethylammonium, hexadecylmethylammonium choline, triethylmethylammonium and diethyldimethylammonium.

The invention also relates to the use of a compound according to the invention as a chromophore.

The term "chromophore" here refers to a molecule whose electronic absorption is at least situated in the spectral range of visible light (between *ca.* 400 nm and 700 nm), which is not exclusive of absorption in the near-UV and near-infrared domains.

As regards molecular chromophores possible applications span from bioimaging purposes, notably as a dye to stain cells, tissues or fibrillar structures like those associated with a variety of neurodegenerative diseases, among which Alzheimer's disease, to the conception of new high performance optical limiter devices relying on non-linear optical properties displayed by compounds objects of the invention.

The invention also relates to a material comprising at least one compound according to the invention.

As it has been previously mentioned, the compounds according to the invention tend to self-assemble and form supramolecular materials.

Said materials can be in the form of crystals, thin films, flakes, platelets or layers and other forms of low-dimensional materials (interfacial films) that may result from self-assembly of chromophores.

Said supramolecular materials can be a pigment, advantageously a pigment of interest for its special optical effect(s).

Accordingly, the invention relates to the use of the compounds (or said supramolecular materials) according to the invention as a pigment or dye.

More particularly, as exemplified in the experimental section, because of their special optical effect(s), said compounds are useful as a luster pigment; even more specifically as a metal effect pigment and/or a pearl luster pigment.

The invention also relates to a metal-like particle that comprises at least one compound according to the invention. Said particle might be embedded within a matrix, including but not limited to polymers, plastics, coating or cosmetic formulation, glass, ceramics, enamels.

The invention also relates to a reflective, photonic, nanophotonic or optoelectronic device, that comprises at least one compound according to the invention.

In the context of the present invention, a reflective device can notably be a solid or liquid mirror.

The term "mirror" here refers to a reflective surface and includes, but is not limited to, a large mirror (e.g. for astronomy or LIDAR technologies), a smart mirror (displaying one or several of the following features: flexibility, transportability, switchability) or an organic-based metal-like liquid film.

The term "organic-based metal-like liquid film" or OMELLF here refers to an organic molecule able to self-assemble to display a reflective surface either at a liquid/liquid interface or at an air/liquid interface by analogy with the reflective surfaces obtained with a low-melting point metal/metal alloy or with an aqueous colloidal preparation of metallic nanoparticles in presence of an appropriate ligand.

The invention also relates to an organic-based metal-like liquid film (OMELLF), or liquid mirror, that comprises at least one compound according to the invention, more specifically a compound comprising one or more C₈-C₂₀ saturated, linear or branched hydrocarbon chain comprising from 8 to 20 carbon atoms, including, but not limited to octyl, decyl, dodecyl, tetradecyl, hexadecyl, octadecyl, eicosyl and the like, within which, in some more particular embodiments, one or more hydrogen atoms are replaced with a halogen atom. Such liquid mirrors can easily be observed at the interface of said compound solution with the air.

Liquid mirrors can be easily achieved notably by simple drop-casting or spraying of pure compound (dissolved in a suitable deposition medium) onto a stirred suitable fluid (e.g. pure water, an aqueous ion-saturated solution, ethylene glycol or another hydrophilic viscous fluid, silicone oil or another hydrophobic viscous fluid, ionic liquids) or by subsequent transfer of such a self-assembled reflective film.

The invention also relates to metal-like reflective coating that comprises at least one compound according to the invention.

Metal-like reflective coatings can be easily achieved notably by simple drop-casting, doctor-blade coating, spin-coating, bar-coating, dip-coating, spraying or vacuum deposition of pure compound dissolved or suspended in a suitable deposition medium.

Reflectance spectra can be recorded with dedicated equipment in order to evaluate quantitatively the efficiency of the thin film to reflect light as a function of wavelength and incidence angle.

The invention also relates to metal-like glittering surface that comprises at least one compound according to the invention.

Metal-like glittering surfaces can be easily achieved notably by incorporating pure compound particles (of variable size) in a non-solubilizing suitable medium (e.g. in a lacquer or in plastics).

The range of application media for the pigments with special optical effects (and in some cases for their constitutive chromophores) according to the invention includes notably paints, coatings, printing inks, cosmetic formulations (e.g. nail lacquers) as well as implementations in the recreational and artistic (e.g. enamel) fields.

Another possible application is the design of pigments for conception of new security inks that enter for example in the fabrication of banknotes, official identity documents, postage stamps, tax banderoles, security labels or product markings.

Another possible application is the design of pigments and/or materials for the conception of new optical reflectors that enter for example in the fabrication of small mirrors for optics/microscopy/interferometry, sensors, very small mirrors for lasers (optical cavity) or mirrors for nomad and transportable devices.

Another possible application is the design of pigments, materials and/or inks for the conception of VCSEL (vertical-cavity surface-emitting laser) or optical waveguides to be included in printed and/or integrated photonic circuits and/or devices, or excitonic nanostructures.

The range of application media for mirror according to the invention includes notably reflective surfaces for the photonic industry, used for example inside equipment for telecommunication applications such as transceivers for data centers. The invention may be also used for larger mirrors used for example in solar electricity production.

The invention also relates to a precursor of a compound of formula (**I**), namely to a compound of following general formula (**Iᵢₙₜ**): 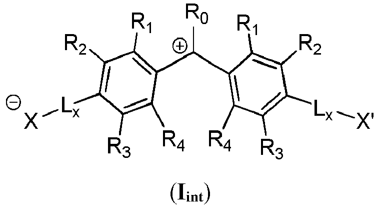 wherein:
▪ X represents an oxygen or a sulfur atom, and:
   - when X represents an oxygen atom, X' represents OH, and
   - when X represents a sulfur atom, X' represents SH; and
▪ R₀ to R₄ and Lₓ are as defined in any of the embodiments related to a compound of formula (**I**).

The invention relates also to the use as dye or pigment of a compound of formula (**Iᵢₙₜ**).

### FIGURES

Figure 1 represents the absorption spectrum of compound **8**-BTMA⁺ in solution in acetonitrile.

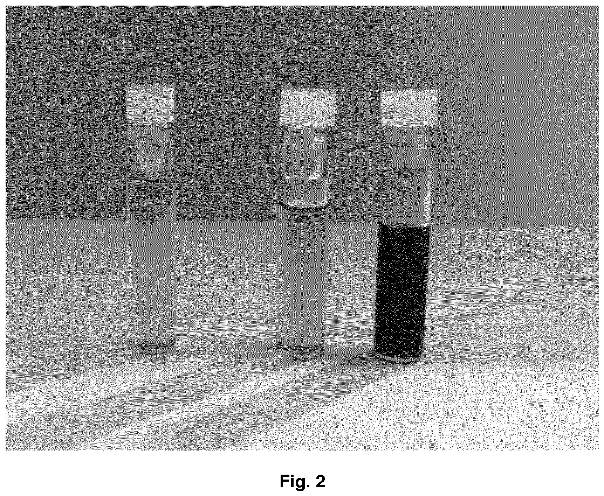

Figure 2 corresponds to a picture of a vivid yellow concentrated solution of compound **8** precursor-2 (left) in methylene chloride, and of a blue colored diluted solution (middle) and of an intense deep blue-purple concentrated solution (right) of compound **8**-BTMA⁺ both in acetonitrile.
Figure 3a corresponds to a picture (outside view) of a blue-purple lustrous thin film obtained on the wall of a glass container after simple evaporation of a solution of **8**-BTMA⁺ in acetonitrile.
Figure 3b corresponds to a picture (inside view) of a blue-purple lustrous thin film obtained on the wall of a glass container after simple evaporation of a solution of **8**-BTMA⁺ in acetonitrile.

The examples that follow illustrate the invention without limiting its scope in any way.

### EXAMPLES

The following abbreviations have been used:
- Ac: : Acetyl (COCH₃)
- Bn: : Benzyl (CH₂Ph)
- Bu: : Butyl (CH₂CH₂CH₂CH₃)
- BTMA: : Benzyltrimethyl ammonium
- ca.: : *circa*
- DDQ: : 2,3-Dichloro-5,6-dicyano-1,4-benzoquinone
- DMF: : Dimethylformamide
- equiv.: : equivalent
- ESI: : Electrospray ionisation
- Et: : Ethyl (CH₂CH₃)
- LA: : Lewis acid
- Me: : Methyl (CH₃)
- MS: : Mass Spectroscopy
- NBS: : *N*-Bromosuccinimide
- NIR: : Near Infra Red
- NMR: : Nuclear Magnetic Resonance
- PPA: : Polyphosphoric acid
- Ph: : Phenyl (C₆H₅)
- Tf₂O: : Triflic anhydride
- TFA: : Trifluoroacetic acid
- THF: : Tetrahydrofuran
- TMEDA: : Tetramethylethylenediamine
- TMSI: : Trimethylsilyl iodide
- vis: : visible
- wt: : weight

### I - Synthesis of the compounds according to the invention

### I-1. General procedures

### ▪ General procedure A:

Compounds of the formula A (persubstituted *para*-quinone methide phenolate) can be prepared by the following Reaction Scheme 1:

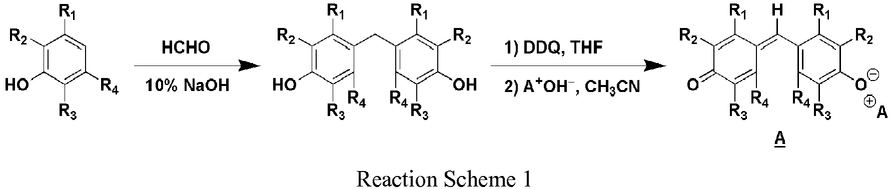

An appropriately 2,3,5,6-tetrasubstituted phenol precursor is engaged under basic conditions in a formaldehyde-mediated dimerization reaction [Burawoi1949]. The resulting diphenol methylene, that often easily crystallizes is then oxidized with DDQ (or another suitable oxidant, which is here more particularly a hydride abstraction reagent) to directly produce the desired quinone methide phenol. The target quinone methide phenolate is subsequently obtained by treatment with a suitable base (preferably an organic hydroxide base [Bukharov1998]).

### ▪ General procedure Abis:

Compounds of the formula A (persubstituted *para*-quinone methide phenolate) can alternatively be prepared by the following Reaction Scheme 1bis:

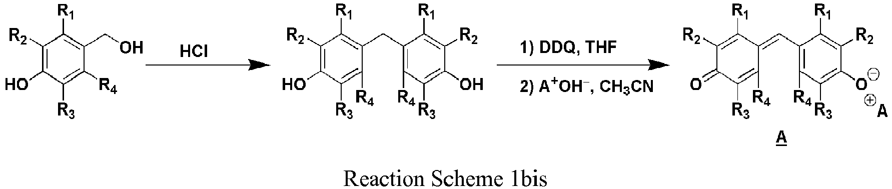

An appropriately 4-(hydroxymethyl)-2,3,5,6-tetrasubstituted phenol precursor is reacted in presence of concentrated hydrochloric acid [Eistert1959]. The resulting diphenol methylene, that often easily crystallizes is then oxidized with DDQ (or another suitable oxidant, which is here more particularly a hydride abstraction reagent) to directly produce the desired quinone methide phenol. The target quinone methide phenolate is finally obtained by treatment with a suitable base (preferably an organic hydroxide base).

### ▪ General procedure Ater:

Compounds of the formula A (persubstituted *para*-quinone methide phenolate) can alternatively be prepared by the following Reaction Scheme 1ter:

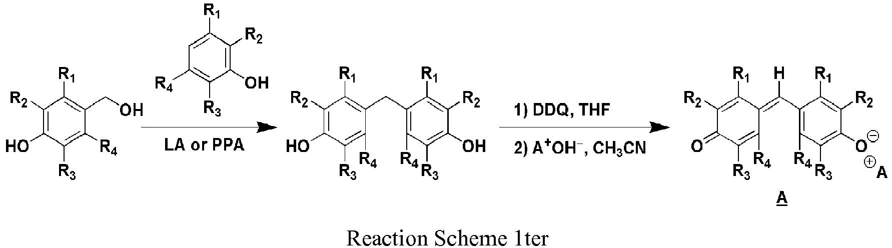

An appropriately 4-(hydroxymethyl)-2,3,5,6-tetrasubstituted phenol precursor is engaged in a Friedel-Crafts reaction (either catalyzed by a Lewis acid or polyphosphoric acid for example) with a 2,3,5,6-tetrasubstituted phenol [Jiang2013] [Balaydin2012]. The resulting diphenol methylene, that often easily crystallizes is then oxidized with DDQ (or another suitable oxidant, which is here more particularly a hydride abstraction reagent) to directly produce the desired quinone methide phenol. The target quinone methide phenolate is subsequently obtained by treatment with a suitable base (preferably an organic hydroxide base).

### ▪ General procedure Aquater:

Compounds of the formula A (persubstituted *para*-quinone methide phenolate) can alternatively be prepared by the following Reaction Scheme 1 quater:

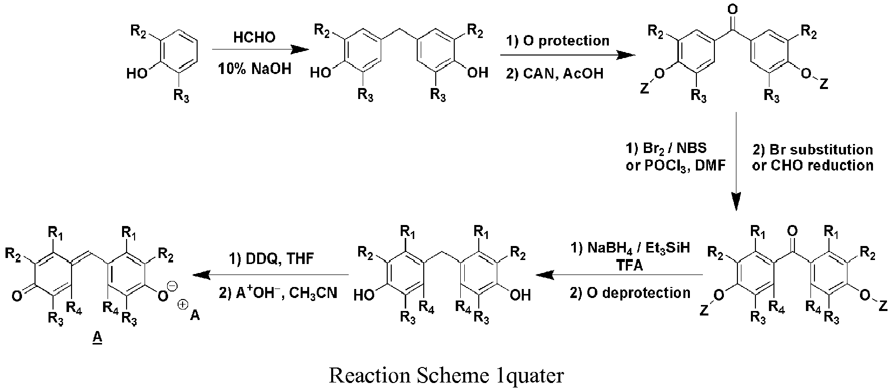

An appropriately 2,6-disubstituted phenol precursor is engaged under basic conditions in a formaldehyde-mediated dimerization reaction [Steelink1968]. The hydroxy groups of the resulting diphenol methylene are then protected, before oxidation of the methylene bridge (by employing cerium ammonium nitrate in acetic acid for example) to give the corresponding benzophenone [Zhou2017]. This diarylketone can then be either halogenated (e.g. brominated with Br₂ or NBS) or formylated (e.g. under Vilsmeier (POCl₃, DMF) or Duff (methenamine, base) conditions) on the vacant 3,5-positions. Either halo or formyl moieties can be interconverted into aryl or alkyl groups e.g. under Suzuki cross-coupling or reductive conditions, respectively. The ketone is then reduced (e.g. by employing NaBH₄ or Et₃SiH in TFA) before a O-deprotection step to recover the phenol moieties. The resulting diphenol methylene can then be oxidized with DDQ (or another suitable oxidant, which is here more particularly a hydride abstraction reagent) to directly produce the desired quinone methide phenol. The target quinone methide phenolate is subsequently obtained by treatment with a suitable base (preferably an organic hydroxide base).

### ▪ General procedure B:

Compounds of the formula B (octasubstituted *para*-thioquinone methide thiophenolate) can be prepared by the following Reaction Scheme 2:

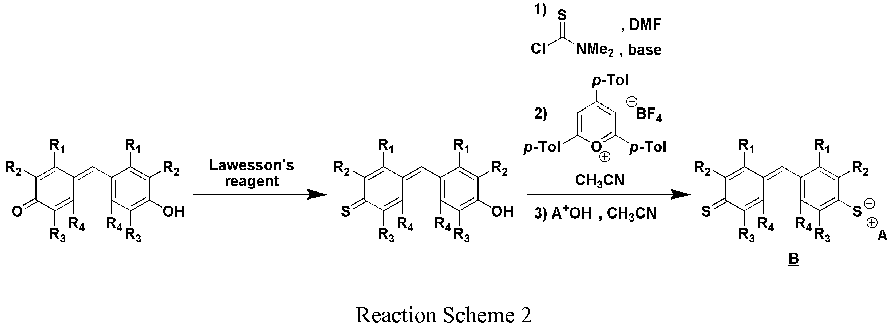

A quinone methide phenol synthesized as described in previous procedures is engaged in a thionation reaction (e.g. by using the Lawesson's reagent) [Vigalok1997]. The corresponding thioquinone methide phenol is then reacted under basic conditions with dimethylthiocarbamoyl chloride. The resulting *O*-aryl *N,N*-dimethylcarbamothioate is then submitted to an ambient-temperature Newman-Kwart rearrangement (e.g. by using an organic photooxidant as catalyst under blue-light irradiation) [Perkowski2015]. The target quinone methide phenolate is subsequently obtained by treatment with a suitable base (preferably an organic hydroxide base).

### ▪ General procedure C:

Compounds of the formula C (octasubstituted aryl-extended *para*-quinone methide phenolate) can be obtained from the key benzophenone (**KB**) precursors prepared by the following Reaction Scheme 3:

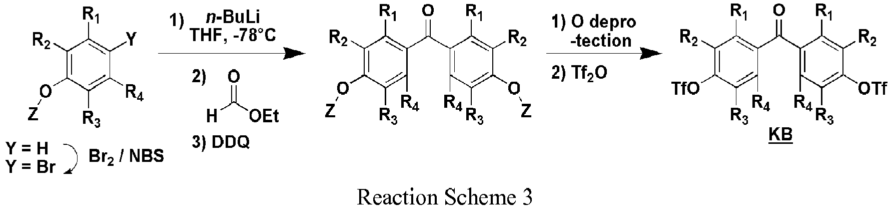

A protected polysubstituted phenol is *para*-brominated according to a classical procedure employing either Br₂ or NBS as reactive species [Zysman2009]. The resulting bromophenoxy precursor is then engaged in a lithiation reaction, and the intermediate is quenched with ethyl formate yielding a carbinol intermediate [Patents] which is subsequently oxidized to the corresponding ketone by using DDQ [Torricelli2013]. The protecting groups are then cleaved (e.g. by using BBr₃ for methoxy groups or TFA for trialkylsilyloxy groups) and the resulting phenol moieties are then reacted with triflic anhydride to give the desired key benzophenone derivatives, KB.

Compounds of the formula C (octasubstituted aryl-extended *para*-quinone methide phenolate) can then be prepared by the following Reaction Scheme 4:

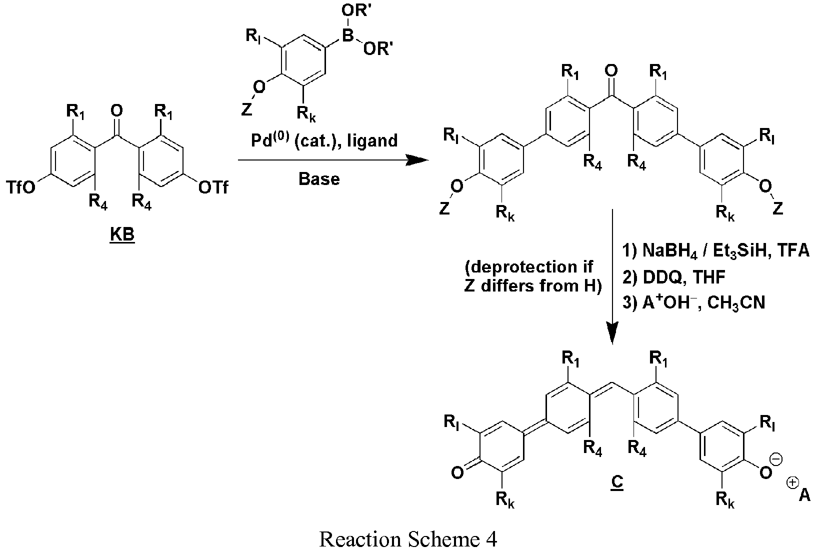

The previously described KB precursors (with R₂ = R₃ = H) may be engaged in a double Suzuki cross-coupling by reacting with an appropriately substituted phenylboronic acid/ester in presence of a base and a palladium catalyst. The resulting extended benzophenone can then be reduced (by either NaBH₄ or Et₃SiH in TFA). After an optional deprotection step, the resulting extended diphenol methylene is O-deprotected if needed and can then be oxidized with DDQ (or another suitable oxidant, which is here more particularly a hydride abstraction reagent) to directly produce the desired extended quinone methide phenol. The target extended quinone methide phenolate is subsequently obtained by treatment with a suitable base (preferably an organic hydroxide base).

### ▪ General procedure Cbis:

Compounds of the formula C (octasubstituted aryl-extended *para*-quinone methide phenolate) can also be obtained from the key methylene (**KM**) precursor prepared by the following Reaction Scheme 5:

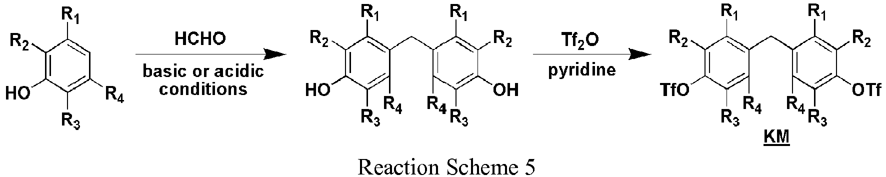

A polysubstituted phenol precursor is engaged under either basic or acidic conditions in a formaldehyde-mediated dimerization reaction [Steelink1968]. The hydroxy groups of the resulting diphenol methylene are subsequently protected by triflate moieties to give the key methylene precursors KM.

Compounds of the formula C (octasubstituted aryl-extended *para*-quinone methide phenolate) can then be prepared by the following Reaction Scheme 4bis:

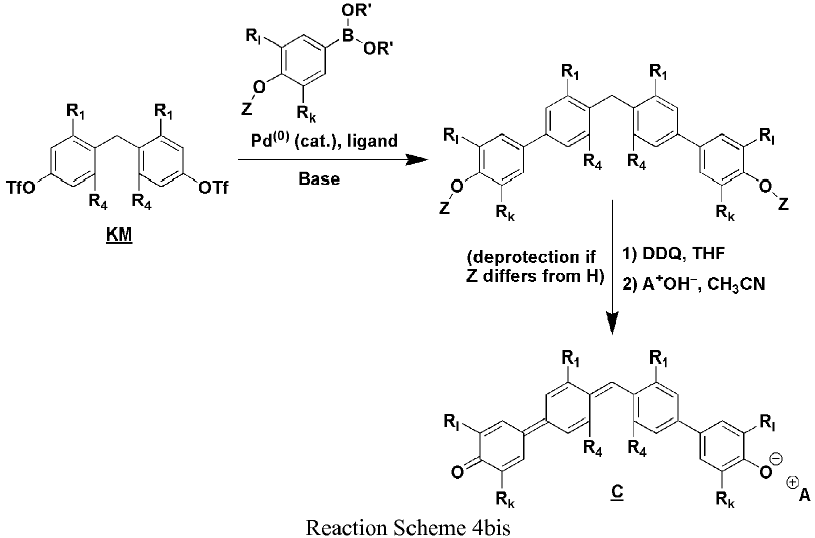

The previously described KM precursors (with R₂ = R₃ = H) may be engaged in a double Suzuki cross-coupling by reacting with an appropriately substituted phenylboronic acid/ester in presence of a base and a palladium catalyst. The resulting extended diphenol methylene is O-deprotected if needed and can then be oxidized with DDQ (or another suitable oxidant, which is here more particularly a hydride abstraction reagent) to directly produce the desired extended quinone methide phenol. The target extended quinone methide phenolate is subsequently obtained by treatment with a suitable base (preferably an organic hydroxide base).

### ▪ General procedure D:

Compounds of the formula D can be obtained from the key benzophenone (KB) precursors prepared by the previously detailed Reaction Scheme 3.

Compounds of the formula D (octasubstituted styryl-extended *para*-quinone methide phenolate) can be prepared by the following Reaction Scheme 6:

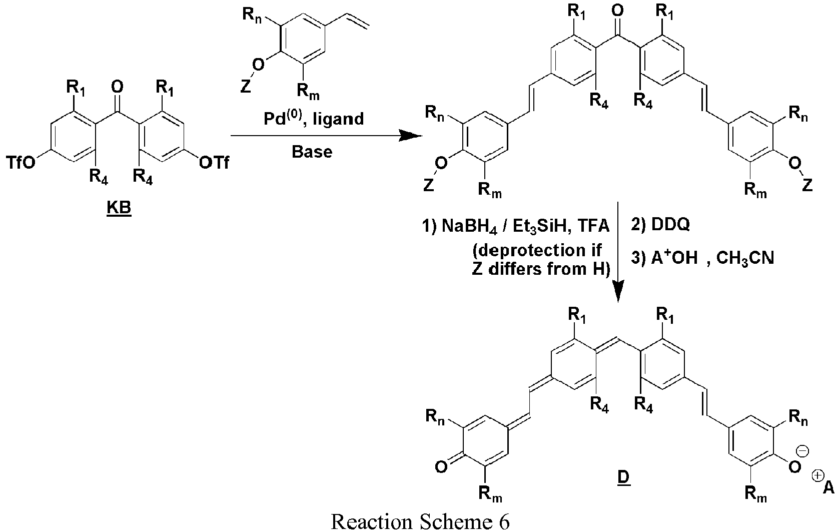

The previously described KB precursors (with R₂ = R₃ = H) may be engaged in a double Heck coupling by reacting with an appropriately substituted styryl precursor in presence of a base and a palladium catalyst. The resulting extended benzophenone can then be reduced (by either NaBH₄ or Et₃SiH in TFA). After an optional deprotection step, the resulting extended diphenol methylene can then be oxidized with DDQ (or another suitable oxidant, which is here more particularly a hydride abstraction reagent) to directly produce the desired styryl-extended quinone methide phenol. The target styryl-extended quinone methide phenolate is subsequently obtained by treatment with a suitable base (preferably an organic hydroxide base).

### ▪ General procedure Dbis:

Compounds of the formula D can also be obtained from the key methylene (KM) precursors prepared by the previously detailed Reaction Scheme 5.

Compounds of the formula D (octasubstituted styryl-extended *para*-quinone methide phenolate) can then be prepared from KM by the following Reaction Scheme 6bis:

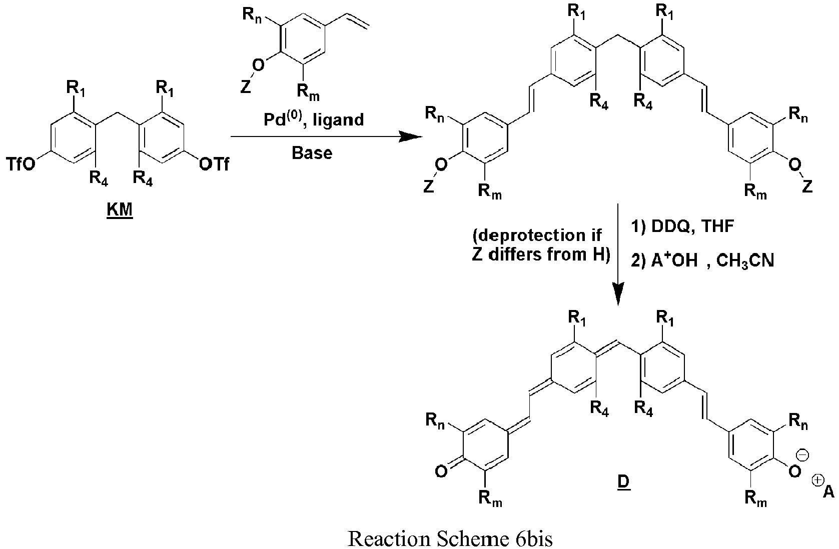

The previously described KM precursor (with R₂ = R₃ = H) may be engaged in a double Heck coupling by reacting with an appropriately substituted styryl precursor in presence of a base and a palladium catalyst. After an optional deprotection step, the resulting extended diphenol methylene can then be oxidized with DDQ (or another suitable oxidant, which is here more particularly a hydride abstraction reagent) to directly produce the desired styryl-extended quinone methide phenol. The target styryl-extended quinone methide phenolate is subsequently obtained by treatment with a suitable base (preferably an organic hydroxide base).

### ▪ General procedure E:

Compounds of the formula E can be obtained from the key benzophenone (KB) precursors prepared by the previously detailed Reaction Scheme 3.

Compounds of the formula E (octasubstituted phenethynyl-extended *para*-quinone methide phenolate) can be prepared by the following Reaction Scheme 7:

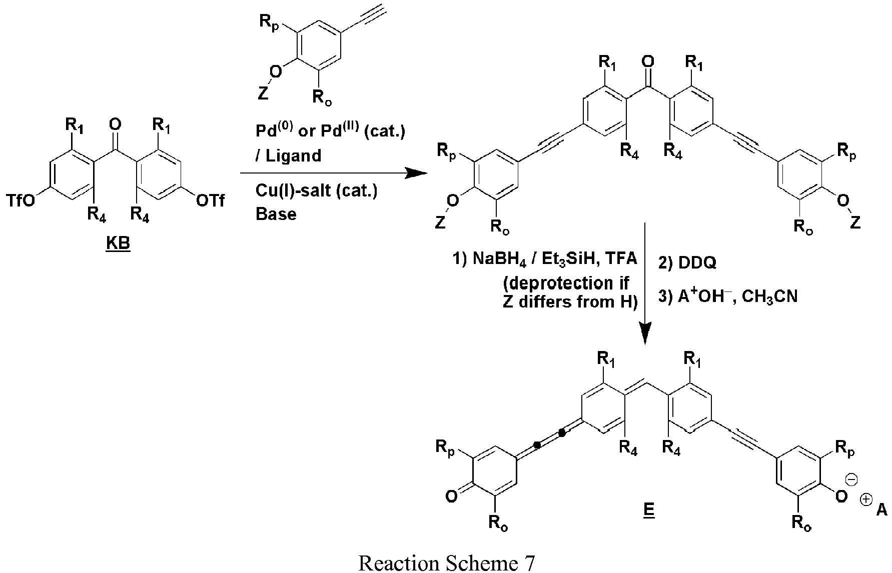

The key benzophenone KB (with R₂ = R₃ = H) may be engaged in a double Sonogashira cross-coupling by reacting with a 3,5-disubstituted-4-hydroxy-ethynylbenzene in presence of a base, Cu(I)-salt and a palladium catalyst. The resulting extended benzophenone can then be reduced (by either NaBH₄ or Et₃SiH in TFA) before an O-deprotection step to recover the phenol moieties. The resulting phenethynyl-extended diphenol methylene can then be oxidized with DDQ (or another suitable oxidant, which is here more particularly a hydride abstraction reagent) to directly produce the desired phenethynyl-extended quinone methide phenol. The target phenethynyl-extended quinone methide phenolate is subsequently obtained by treatment with a suitable base (preferably an organic hydroxide base).

### ▪ General procedure Ebis:

Compounds of the formula G can also be obtained from the key methylene (KM) precursor prepared by the previously detailed Reaction Scheme 5.

Compounds of the formula G (octasubstituted phenethynyl-extended *para*-quinone methide phenolate) can be prepared by the following Reaction Scheme 7bis:

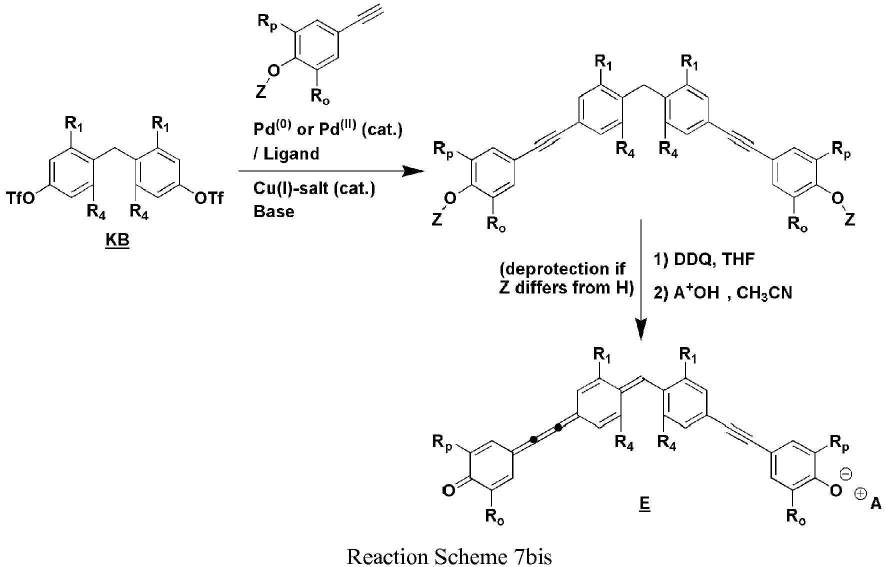

The key methylene KM (with R₂ = R₃ = H) may be engaged in a double Sonogashira cross-coupling by reacting with a 3,5-disubstituted-4-hydroxy-ethynylbenzene in presence of a base, Cu(I)-salt and a palladium catalyst. The resulting phenethynyl-extended diphenol methylene can then be oxidized with DDQ (or another suitable oxidant, which is here more particularly a hydride abstraction reagent) to directly produce the desired phenethynyl-extended quinone methide phenol. The target phenethynyl-extended quinone methide phenolate is subsequently obtained by treatment with a suitable base (preferably an organic hydroxide base).

### ▪ General procedure F:

Compounds of the formula H (persubstituted bridge-arylated para-quinone methide phenolate) can be prepared by the following Reaction Scheme 8:

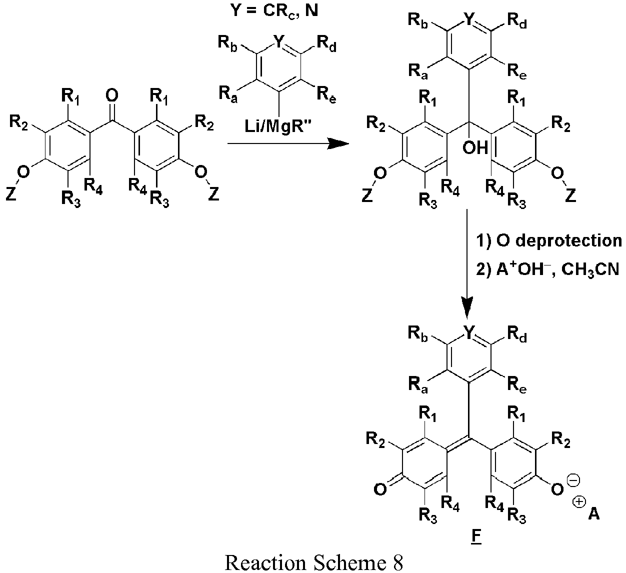

A persubstituted benzophenone bearing two protected phenol moieties in *para*-positions (obtained for example from the procedures previously described in Reaction Schemes 1quater or 3) may be engaged in presence of an aryl organometallics [Harrer1975]. The tertiary alcohol intermediate thus obtained is engaged in a O-deprotection step to directly produce the desired bridged-arylated quinone methide phenol to directly produce the desired bridged-arylated quinone methide phenol. If not, a subsequent oxidation step with DDQ (or another suitable oxidant, which is here more particularly a hydride abstraction reagent) may be performed. The target bridge-arylated quinone methide phenolate is subsequently obtained by treatment with a suitable base (preferably an organic hydroxide base).

### ▪ General procedure Fbis:

Compounds of the formula F (persubstituted bridge-arylated *para*-quinone methide phenolate) can be prepared by the following Reaction Scheme 8bis:

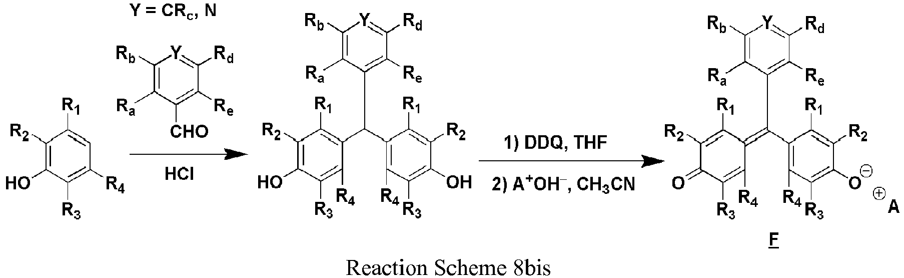

A 2,3,5,6-tetrasubstituted phenol precursor is engaged in a dimerization in presence of a formylated aryl [Yamada1989]. The resulting bridge-arylated diphenol methylene can then be oxidized with DDQ (or another suitable oxidant, which is here more particularly a hydride abstraction reagent) to directly produce the desired bridged quinone methide phenol. The target bridge-arylated quinone methide phenolate is subsequently obtained by treatment with a suitable base (preferably an organic hydroxide base).

### ▪ General procedure G:

Compounds of the formula G (SiR₂-bridged persubstituted *para*-quinone methide phenolate) can be prepared by the following Reaction Scheme 9:

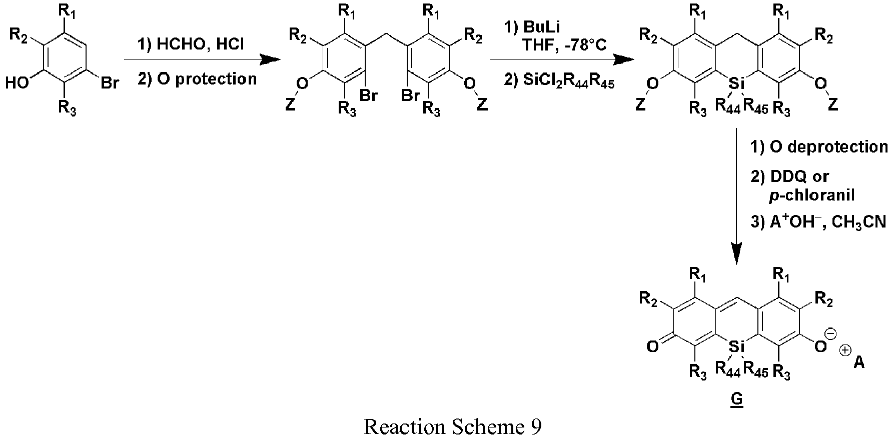

A *meta*-brominated trisubstituted phenol precursor is engaged in a formaldehyde-mediated dimerization reaction to give the corresponding dibromo diphenol methylene [Best2013]. The hydroxyl moieties are then protected and bromine atoms are exchanged in presence of BuLi in order to produce the corresponding dilithium intermediate which is quenched by addition of a disubstituted silicon dichloride reagent [Best2013]. After a O-deprotection step, the resulting SiR₂-bridged diphenol methylene is then oxidized with DDQ or *p*-chloranil (or another suitable oxidant, which is here more particularly a hydride abstraction reagent) to directly produce the desired SiR₂-bridged quinone methide phenol. The target SiR₂-bridged quinone methide phenolate is subsequently obtained by treatment with a suitable base (preferably an organic hydroxide base).

### ▪ General procedure H:

Compounds of the formula H (CMe₂-bridged *ortho*-substituted diphenylcarbenium) can be prepared by the following Reaction Scheme 10:

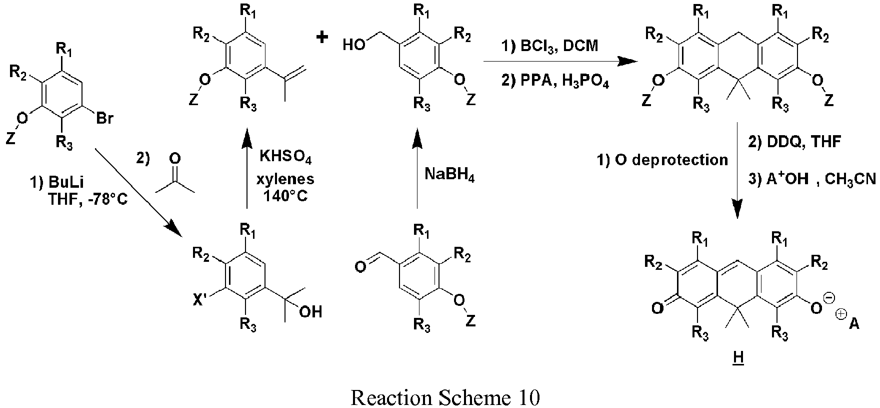

A protected *meta*-brominated trisubstituted phenol precursor is engaged in a halogen-metal exchange reaction to produce the corresponding lithiated intermediate which is quenched by addition of dry acetone. The resulting tertiary alcohol is then dehydrated by heating in presence of KHSO₄ to give a methylene exo compound. The latter molecule is engaged with a closely related counterpart (but bearing a benzylic alcohol moiety) in a sequence of reactions allowing coupling and bridging of these two parts [Pastierik2014]. After a O-deprotection step, the resulting CMe₂-bridged diphenol methylene is then oxidized with DDQ or *p*-chloranil (or another suitable oxidant, which is here more particularly a hydride abstraction reagent) to directly produce the desired CMe₂-bridged quinone methide phenol. The target CMe₂-bridged quinone methide phenolate is subsequently obtained by treatment with a suitable base (preferably an organic hydroxide base).

### ▪ General procedure Hbis:

Compounds of the formula H (CMe₂-bridged *ortho*-substituted diphenylcarbenium) can be prepared by the following Reaction Scheme 10bis:

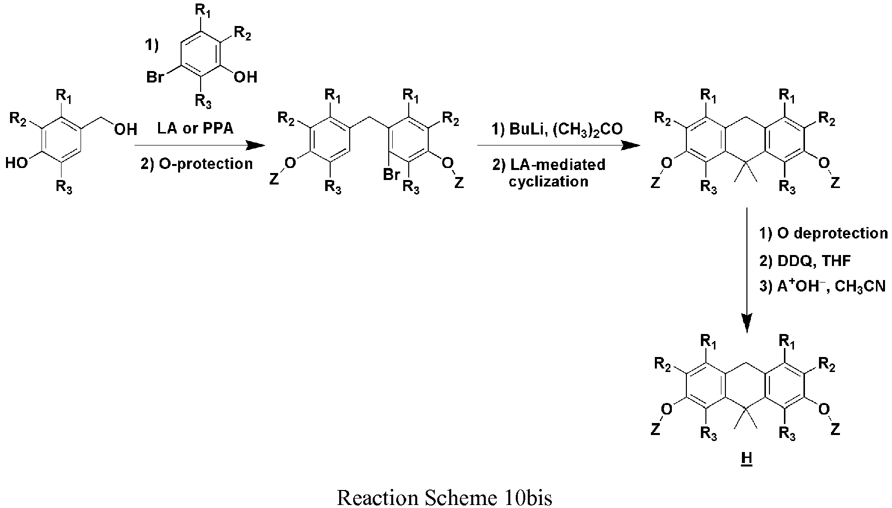

A *para*-hydroxy trisubstituted benzyl alcohol precursor is engaged in a Lewis acid-mediated dimerization with an appropriate *meta*-bromo trisubstituted phenol to give a bromo diphenol methylene which hydroxyl groups are subsequently protected. A halogen-metal exchange reaction step then allows production of the corresponding lithiated intermediate which is quenched by addition of dry acetone. The resulting tertiary alcohol is then dehydrated by heating in presence of KHSO₄ to give a methylene exo compound that allows access to the corresponding CMe₂-bridged methylene after a Lewis acid-mediated cyclization step. After a O-deprotection step, the resulting CMe₂-bridged diphenol methylene is then oxidized with DDQ or *p*-chloranil (or another suitable oxidant, which is here more particularly a hydride abstraction reagent) to directly produce the desired CMe₂-bridged quinone methide phenol. The target CMe₂-bridged quinone methide phenolate is subsequently obtained by treatment with a suitable base (preferably an organic hydroxide base).

### ▪ General procedure I:

Compounds of the formula I (three and more atoms-bridged persubstituted para-quinone methide phenolate) can be prepared by the following Reaction Scheme 11:

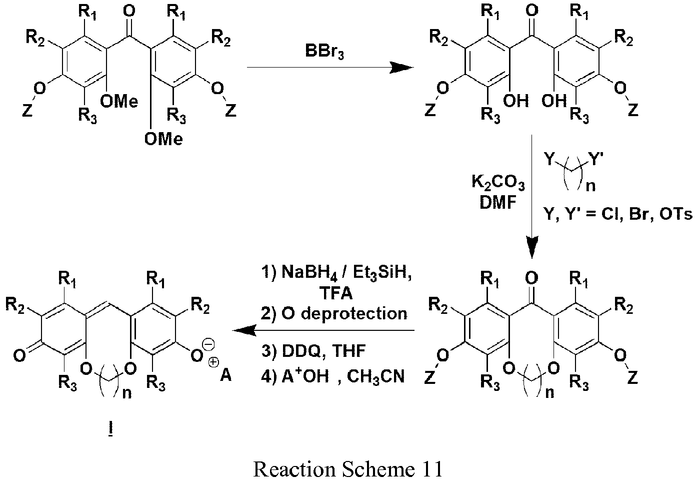

A persubstituted benzophenone bearing two protected phenol moieties in *para*-positions and two phenol moieties in *ortho*-positions (obtained for example from the procedures previously described in Reaction Schemes 1quater or 3) may be engaged in a bis-etherification. If R₄ = OMe on the starting benzophenone a prior step of demethylation is necessary, employing either BBr₃ or TMSI as reactive species. The free phenol moieties are then bridged together through an aliphatic chain of length controlled by the nature of the reagent employed (e.g.: CH₂BrCl for n = 1, TsO-(CH₂)₂-OTs for n = 2) [Sorrell1997]. The resulting bridged benzophenone is then engaged in a reduction to methylene (e.g. by employing NaBH₄ or Et₃SiH in TFA) followed by a deprotection step to recover the phenol moieties. The resulting bridged diphenol methylene can then be oxidized with DDQ (or another suitable oxidant, which is here more particularly a hydride abstraction reagent) to directly produce the desired bridged quinone methide phenol. The target bridged quinone methide phenolate is subsequently obtained by treatment with a suitable base (preferably an organic hydroxide base).

### ▪ General procedure Ibis:

Compounds of the formula I (three and more atoms-bridged persubstituted *para*-quinone methide phenolate) can alternatively be prepared by the following Reaction Scheme 11bis:

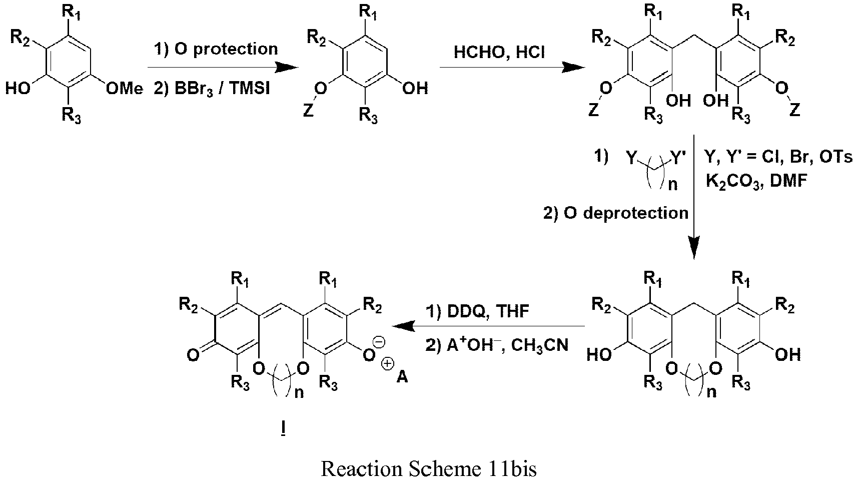

A *meta*-methoxylated trisubstituted phenol precursor is submitted to an O-protection step before being engaged in the presence of a demethylating agent (e.g. BBr₃ or TMSI) to give the corresponding phenol. The resulting compound is then dimerized at its *ortho* position, in the presence of formaldehyde under acidic conditions (notably HCl, H₂SO₄ or AcOH), to give a diphenol methylene. The hydroxyl groups are then bridged together through an aliphatic chain of length controlled by the nature of the reagent employed (e.g.: CH₂BrCl for n = 1, TsO-(CH₂)₂-OTs for n = 2) [Sorre111997]. The resulting bridged methylene is submitted to an O-deprotection step to allow recovering of the *para*-phenol moieties and can then be oxidized with DDQ (or another suitable oxidant, which is here more particularly a hydride abstraction reagent) to directly produce the desired bridged quinone methide phenol. The target bridged quinone methide phenolate is subsequently obtained by treatment with a suitable base (preferably an organic hydroxide base).

### ▪ General procedure J:

Compounds of the formula J (bridge-alkylated persubstituted *para*-quinone methide phenolate) can be prepared by the following Reaction Scheme 12:

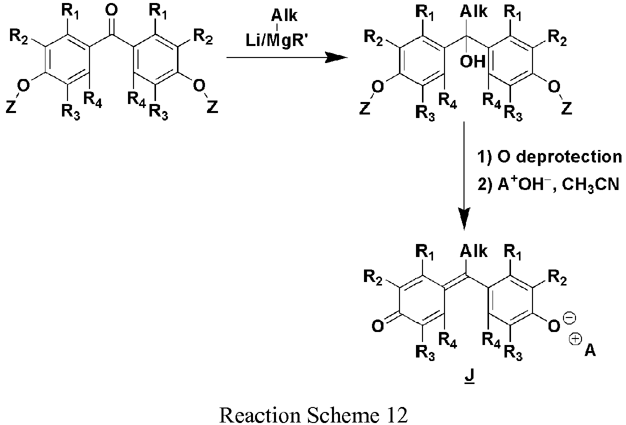

A persubstituted benzophenone bearing two protected phenol moieties (obtained for example from the procedures previously described in Reaction Schemes 1quater or 3) may be engaged in presence of an alkyl (either linear, branched or cyclic) organometallics [Kirstel981]. The tertiary alcohol intermediate thus obtained is engaged in a O-deprotection step to directly produce the desired bridge-alkylated quinone methide phenol. If not, a subsequent oxidation step with DDQ (or another suitable oxidant, which is here more particularly a hydride abstraction reagent) may be performed. The target bridge-alkylated quinone methide phenolate is subsequently obtained by treatment with a suitable base (preferably an organic hydroxide base).

### ▪ General procedure Jbis:

Compounds of the formula J (bridge-alkylated persubstituted *para*-quinone methide phenolate) can be prepared by the following Reaction Scheme 12bis:

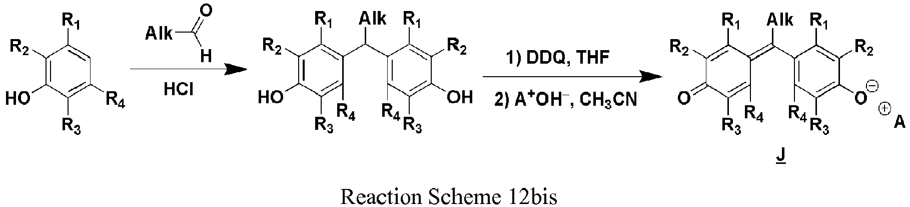

A 2,3,5,6-tetrasubstituted phenol precursor is engaged in a dimerization in presence of an alkanal [Singh2014]. The resulting bridge-alkylated methylene can then be oxidized with DDQ (or another suitable oxidant, which is here more particularly a hydride abstraction reagent) to directly produce the desired bridge-alkylated quinone methide phenol. The target bridge-alkylated quinone methide phenolate is subsequently obtained by treatment with a suitable base (preferably an organic hydroxide base).

### ▪ General procedure Jter:

Compounds of the formula J (bridge-alkylated persubstituted *para*-quinone methide phenolate) can be prepared by the following Reaction Scheme 12ter:

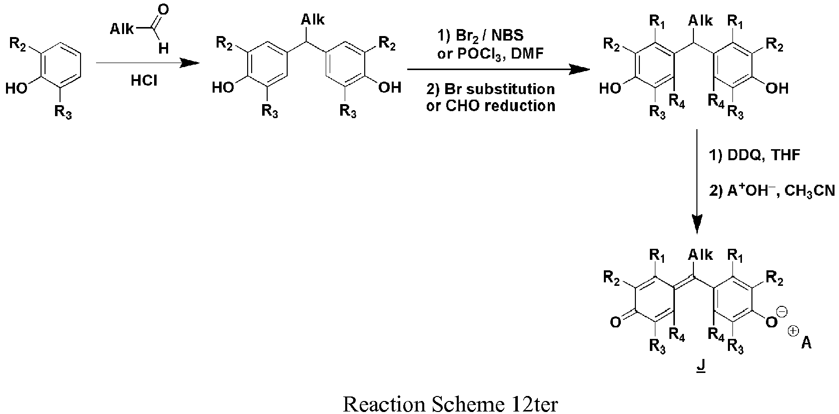

A 2,6-disubstituted phenol precursor is engaged in a dimerization reaction in presence of an alkanal [Singh2014]. The resulting bridge-alkylated diphenol methylene can then be either halogenated (e.g. brominated with Br₂ or NBS) or formylated (e.g. under Vilsmeier (POCl₃, DMF) or Duff (methenamine, base) conditions) on the vacant 3,5 positions. Either halo or formyl moieties can be interconverted into aryl or alkyl groups e.g. under, respectively, Suzuki cross-coupling or reductive conditions. The resulting bridge-alkylated persubstituted diphenol methylene can then be oxidized with DDQ (or another suitable oxidant, which is here more particularly a hydride abstraction reagent) to directly produce the desired bridge-alkylated quinone methide phenol. The target bridge-alkylated quinone methide phenolate is subsequently obtained by treatment with a suitable base (preferably an organic hydroxide base).

### ▪ General procedure K:

Compounds of the formula K (cyano-bridge persubstituted *para*-quinone methide phenolate) can be prepared by the following Reaction Scheme 13.

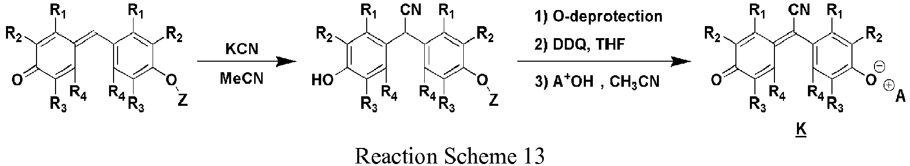

A persubstituted quinone methide phenol compound (previously O-protected) is engaged in a cyanation reaction, employing either KCN or another cyanide salt. The resulting cyano methylene compound can then be deprotected and oxidized with DDQ (or another suitable oxidant, which is here more particularly a hydride abstraction reagent) to produce the corresponding bridge-cyanated quinone methide phenol. The target bridge-cyanated quinone methide phenolate is subsequently obtained by treatment with a suitable base (preferably an organic hydroxide base).

### ▪ General procedure L:

Compounds of the formula L (amino-bridge persubstituted *para*-quinone methide phenolate) can be prepared by the following Reaction Scheme 14:

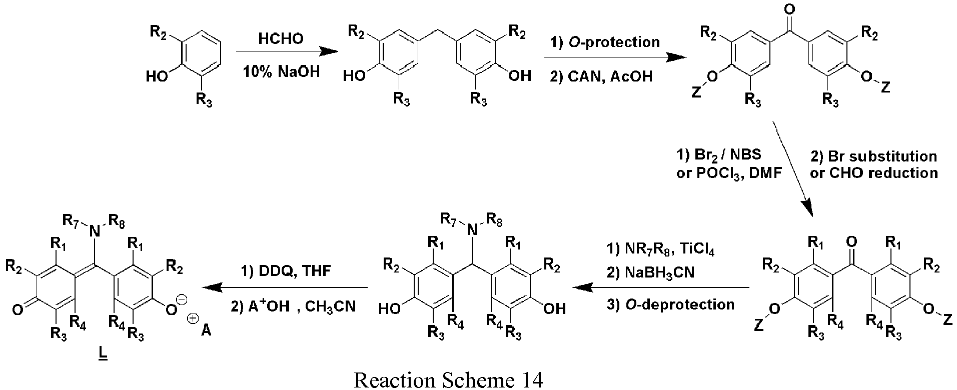

An appropriately 2,6-disubstituted phenol precursor is engaged under basic conditions in a formaldehyde-mediated dimerization reaction [Steelink1968]. The hydroxy groups of the resulting diphenol methylene are then protected, before oxidation of the methylene bridge (by employing cerium ammonium nitrate in acetic acid for example) to give the corresponding benzophenone [Zhou2017]. This diarylketone can then be either halogenated (e.g. brominated with Br₂ or NBS) or formylated (e.g. under Vilsmeier (POCl₃, DMF) or Duff (methenamine, base) conditions) on the vacant 3,5-positions. Either halo or formyl moieties can be interconverted into aryl or alkyl groups, e.g. under Suzuki cross-coupling or reductive conditions, respectively. The ketone is then submitted to a reductive amination procedure (e.g. by employing TiCl₄ in the first step and NaBH₃CN in the second one) [Rahman2004] before a O-deprotection step to recover the phenol moieties. The resulting amino-bridge diphenol methylene can then be oxidized with DDQ (or another suitable oxidant, which is here more particularly a hydride abstraction reagent) to directly produce the desired quinone methide phenol. The target quinone methide phenolate is subsequently obtained by treatment with a suitable base (preferably an organic hydroxide base).

### ▪ References cited in the above described general procedures:

[Burawoi1949]: Burawoi, A., Chamberlain, J. T. J. Chem. Soc., 1949, 624-626.
[Bukharov1998]: Bukharov, C. V., Pod"yachev, S. N., Syakaev, V. V., Zgadzai, O. E., Russ. J. Gen. Chem., 1998, 68 (3), 429-434.
[Eistert1959]: Eistert, B., Bock, G. Chem. Berichte, 1959, 92, 1247-1257.
[Jiang2013]: Jiang, B., Guo, S., Shi, D., Guo, C., Wang, T. Eur. J. Med. Chem., 2013, 64, 129-136.
[Balaydin2012]: Balaydin, H.T., Soyut, H., Ekinci, D., Göksu, S., Beydemir, S., Menzek, A., 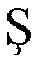 ahin, E. *J. Enzyme Inhib. Med. Chem.,* **2012,** 27(1), 43-50.
[Steelink1968]: Steelink, C., Fitzpatrick, J. D., Kispert, L. D., Hyde, J. S. J. Am. Chem. Soc., 1968, 90, 4354-4361.
[Zhou2017]: Zhou, Y., Mukherjee, M., Gupta, A. K., Wulff, W. D. Org. Lett., 2017, 19, 2230-2233.
[Vigalok1997]: Vigalok, A., Milstein, D. J. Am. Chem. Soc., 1997, 119, 7873-7874.
[Perkowski2015]: Perkowski, A. J., Cruz, C. L., Nicewicz, D. A., J. Am. Chem. Soc., 2015, 137, 15684-15687].
[Zysman2009]: Zysman-Colman, E., Arias, K., Siegel, J. S. Can. J. Chem., 2009, 87, 440-447.
[Patents]: CA 2311064; US 6670512.
[Torricelli2013]: Torricelli, F., Bosson, J., Besnard, C., Chekini, M., Bürgi, T., Lacour, J. Angew. Chem. Int. Ed. 2013, 52, 1796-1800.
[Harrer1975]: Harrer, W., Kurreck, H., Reusch, J., Gierke, W. Tetrahedron, 1975, 31, 625-632.
[Yamada1989]: Yamada, F.; Nishiyama, T.; Yamamoto, M.; Tanaka, K. Bull. Chem. Soc. Jpn. 1989, 62, 3603-3608.
[Best2013]: Best, Q. A., Sattenapally, N., Dyer, D. J., Scott, C. N., McCarroll, M. E. J. Am. Chem. Soc. 2013, 135, 13365-13370.
[Pastierik2014]: Pastierik, T., Šebej, P., Medalová, J., Štacko, P., Klán, P. J. Org. Chem. 2014, 79, 3374-3382.
[Sorrell1997]: Sorrell, T. N., Yuan, H. J. Org. Chem. 1997, 62, 1899-1902.
[Kirste1981]: Kirste, B., Harrer, W., Kurreck, H., Schubert, K., Bauer, H., Gierke, W. J. Am. Chem. Soc. 1981, 103, 6280-6286.
[Singh2014]: Singh, D., Chaudhari, U. V., Deota P. T. Tetrahedron, 2014, 70, 4485-4493.
[Rahman2004]: Rahman, O., Kihlberg, T., Långström, B. Org. Biomol. Chem. 2004, 2, 1612-1616.

### I-2. Examples of syntheses of compounds according to the invention

### I-2.i. Synthesis of compound 8 according to general procedure A

Compound **8** precursor-1: 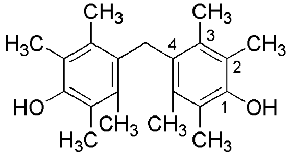 4,4' -methylenebis(2,3,5,6-tetramethylphenol)

To a warm solution of 2,3,5,6-tetramethylphenol (durenol) [Diemer2006] (0.44 g, 2.93 mmol) in 10 mL of 10% sodium hydroxide, was added 2.2 mL of 37% formaldehyde. After completion of the reaction (monitored by TLC ; conditions: CH₂Cl₂ 100%), the reaction mixture was poured into a saturated aqueous solution of NH₄Cl, CH₂Cl₂ was added several times, the organic phases were gathered, dried on MgSO₄ and evaporated in vacuo to yield a colorless solid (0.21 g, 46%) that may be recrystallized from ethanol. [partial characterization in [Burawoy1949] and [Kehse1974]]. ¹H NMR (400 MHz, CDCl₃): *δ* 4.50 (s, D₂O exch., 2H, OH); 4.08 (s, 2H, CH₂); 2.15 (s, 12H, ²CH₃); 2.02 (s, 12H, ³CH₃). ¹³C NMR (100 MHz, CDCl₃): *δ* 149.5 (C₁); 133.2 (C₃); 131.5 (C₄); 119.2 (C₂); 32.7 (CH₂); 12.3 (²CH₃); 12.1 (³CH₃). ESI-HRMS: *m*/*z*: calculated for C₂₁H₂₈O₂Na: 335.1982 [M+Na]⁺; found 335.2556 [M+Na]⁺.

### ▪ Compound 8 precursor-2:

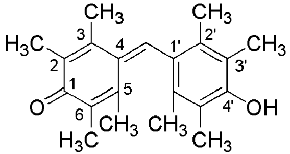

### 2,3,5,6-Tetramethyl-4-(2,3,5,6-tetramethyl-4-hydroxy-benzylidene) cyclohexa-2,5-dienone

To a clear yellow solution of 4,4'-methylenebis(2,3,5,6-tetramethylphenol) (0.174 g, 0.56 mmol) in 10 mL of THF, was added dropwise a solution of DDQ (0.133 g, 0.56 mmol) in 10 mL of THF, the reaction medium turns blue immediately and brightens progressively. After 2 h of stirring at room temperature the resulting light orange solution is washed first with a saturated aqueous solution of NH₄Cl, then with brine. Methylene chloride is added and the organic phases gathered, dried on MgSO₄ and evaporated in vacuo to yield an orange/yellow solid which is purified via the Biotage Isolera One (silica-packed snap cartridge; 0-50% CH₂Cl₂/cyclohexane) to afford a yellow microcristalline solid (0.026 g, 15%). ¹H NMR (600 MHz, CDCl₃): *δ* 7.39 (=CH); 4.79 (s, D₂O exch., 1H, OH); 2.32 (s, 3H, ^{3/5}CH₃); 2.19 (s, 6H, ^{3'}CH₃); 2.0 (m, 9H, ^{2'}CH₃ + ^{2/6}CH₃); 1.92 (s, 3H, ^{2/6}CH₃), 1.52 (s, 3H, ^{3/5}CH₃). ¹³C NMR (150 MHz, CDCl₃): *δ* 186.0 (C₁); 151.7 (C_{4'}); 143.5 (C_{3/5}); 143.4 (C_{3/5}); 139.7 (=CH); 137.5 (C₄); 133.8 (C_{2/6}); 131.2 (C₁); 130.6 (C_{2/6}); 119.7 (C_{3'}); 18.4 (^{3/5} CH₃); 16.7 (^{3/5}CH₃); 12.5 (^{2/6}CH₃); 12.4 (^{2'}CH₃); 12.3 (^{3'}CH₃); 12.1 (^{2/6}CH₃). ESI-HRMS: *m*/*z*: calculated for C₂₁H₂₇O₂: 311.2006 [M+H]⁺; found 311.1995 [M+H]⁺.

### ▪ Compound 8-BTMA⁺:

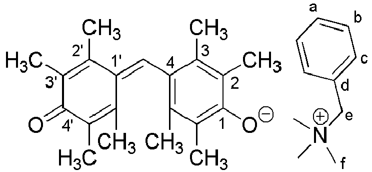

### Benzyltrimethylammonium 2,3,5, 6-tetramethyl-4-((2,3,5,6-tetramethyl-4-oxocyclohexa-2,5-dienylidene)methyl)phenolate

To a clear yellow solution of 2,3,5,6-tetramethyl-4-(2,3,5,6-tetramethyl-4-hydroxybenzylidene)cyclohexa-2,5-dienone (20 mg, 0.064 mmol) in 10 mL of CH₃CN, was dropwise added a benzyltrimethylammonium hydroxide solution (Triton B) (40% in methanol, 0.035 mL, 0.077 mmol), the reaction medium turns dark purple immediately. After 30 min of stirring at room temperature the reaction mixture is concentrated under reduced pressure and the residue taken up in CH₂Cl₂. The organic layer is washed with a small amount of distilled water (to get rid off excess Triton B) and evaporated in vacuo to afford a lustrous purple lacquer in a quantitative yield (29 mg, 98%). ¹H NMR (400 MHz, CDCl₃): *δ* 7.55-7.51 (m, 2H, H_{c}); 7.47-7.38 (m, 4H, H_{b} + Hₐ + =CH); 4.71 (s, 2H, N⁺CH₂); 3.22 (s, 9H, N⁺CH₃); 2.03 (s, 9H, ²CH₃); 2.00 (s, 6H, ^{3'}CH₃); 1.93 (s, 9H, ³CH₃). ¹³C NMR (100 MHz, CDCl₃): *δ* 185.3 (C_{4'}); 169.3 (C₁); 140.5 (=CH); 137.7; 137.1; 133.5; 133.0 (C_{c}); 130.9 (Cₐ); 129.3 (C_{b}); 127.6 (C_{d}); 126.4; 126.3; 115.1; 114.5; 69.3 (N⁺CH₂); 52.7 (N⁺CH₃); 17.5 (CH₃); 12.3 (CH₃). HRMS (ESI-): *m*/*z*:
calculated for C₂₁H₂₅O₂: 309.1860 [M-BTMA]⁻; found 309.1825 [M-BTMA]⁻. HRMS (ESI+): *m*/*z*: calculated for C₁₀H₁₆N: 150.1277 [BTMA]⁺; found 150.3736 [BTMA]⁺.
▪ **References** reporting the preparation and characterization of some starting materials used in the above described general:
[Diemer2006] Diemer, V., Chaumeil, H., Defoin, A., Fort, A., Boeglin, A., Carré, C. Eur. J. Org. Chem., 2006, 2727-2738.
[Burawoy1949] Burawoy, A., Chamberlain, J. T. J. Chem. Soc., 1949, 624-626.
[Kehse1974] Kehse, K., Kawerau, H. G. Arch. Pharm., 1974, 307 (12), 934-942.

### II - Optical properties of the compounds according to the invention

### II-1. Absorption properties

Absorbance of compound **8**-BTMA⁺ was measured in a 1 cm optical path quartz cuvette (against reference 1 cm optical path quartz cuvette containing pure acetonitrile) in a double-beam Cary 500 spectrophotometer (Varian).

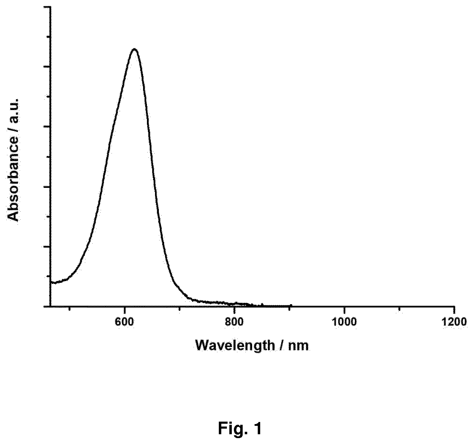

As it appears on Figure 1, the compound according to the invention is characterized by a single absorption band in the visible region of the electromagnetic spectrum.

Accordingly, chromophoric properties of compounds of the invention or of their precursor of neutral charge of formula (**Iᵢₙₜ**) as mentioned in the above description are directly observed from these solutions. For example, a vivid yellow color is observed for **8** precursor-2, dissolved in methylene chloride and an intense deep blue-purple color is observed for **8**-BTMA⁺, dissolved in acetonitrile.

It's worth noting that solution colors of the anionic quinone methides are somewhat affected by the nature of either the solvent, the cation size or dilution (ion pair association) as observed for **8**-A⁺.

### II-2. Other optical properties

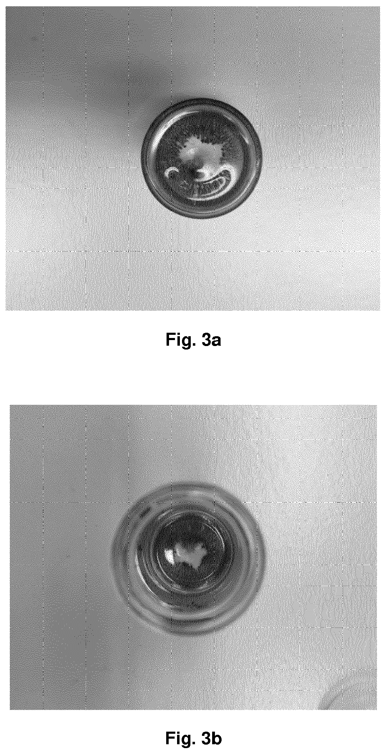

Figures 3a & 3b corresponds to pictures of a blue-purple lustrous thin film obtained on the wall of a container after simple evaporation of a solution of **8**-BTMA⁺ in acetonitrile. Such luster effects are characteristic of chromophores displaying a single sharp absorption band in the visible region of the electromagnetic spectrum, and a high molar extinction coefficient, as the compounds according to the invention.

### III - Films made of compounds according to the invention

### III-1. Elaboration and characterization of solid films made of compounds of the invention: spin coating and quantitative reflectance measurements

The compounds according to the invention are soluble in most organic (polar and non polar) solvents. For example, an acetonitrile (CH₃CN) solution of a compound of the invention (for example at a concentration of 5 mg/mL) can be prepared.

This solution is spin-coated onto a substrate, and after solvent evaporation under ambient conditions, a uniform film exhibiting a reflective appearance is obtained.

Films of different thicknesses can be spin-coated by varying the spin-coating speed or acceleration or duration, or by varying the concentration or the volume of the solution that is deposited onto the substrate. Also, several layers of films can be superimposed by repeating successive steps of spin coating as above.

Drop-casting, dip-coating, spraying, bar-coating and doctor blade techniques may also represent alternative efficient modes of preparation of solid films made of compounds of the invention. Optical properties are somewhat angle-dependent and reinforced by the background color of the substrate.

### III-2. Elaboration and characterization of organic metal-like liquid films made of compounds of the invention: dropwise addition and quantitative reflectance measurements

Whether the compound of interest bears rather hydrophilic or lipophilic moieties, it is mandatory to appropriately choose the nature of the deposition fluid in order to obtain a film with optimized optical properties.

A solution of one of the compounds (around 10mg/mL or more) is dropwise added into a stirred saturated salt solution (e.g. ammonium salt). In condition that the compound is deposited on an appropriate liquid, a homogeneous reflective film covers the liquid surface, that can if needed be carefully transferred onto another surface.

Spraying or more sophisticated techniques may be envisioned to perform an efficient mode of preparation of films made of compounds of the invention (regarding the purpose or the type of liquid on which deposit).

## Claims

1. A compound of following general formula (**I**): 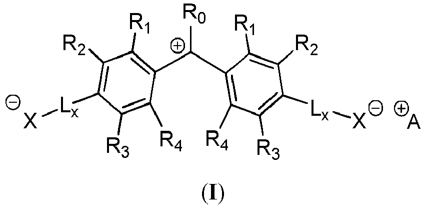 wherein:
▪ X represents an oxygen or a sulfur atom;
▪ **R**₀ represents a hydrogen atom, a halogen atom, a (C₁-C₆)alkyl, (C₁-C₆)haloalkyl, aryl-(C₁-C₆)alkyl, heteroaryl-(C₁-C₆)alkyl, cycloalkyl, cycloalkyl-(C₁-C₆)alkyl, heterocycle, heterocycle-(C₁-C₆)alkyl, OR₅, SR₆, NR₇R₈, COR₁₁, CO₂R₁₂, CONR₁₃R₁₄, SO₂R₁₅, CN or NO₂ group, or is selected from the group consisting of: 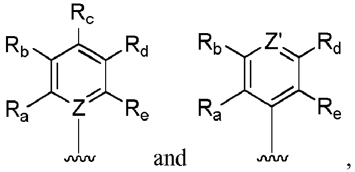 wherein:
- Z represents C or N⁺ A_{z}⁻ and Z' represents N or N⁺-R_{c}' A_{z}⁻, wherein
A_{z}⁻ represents a monovalent organic or inorganic anion, and
R_{c}' represents a (C₁-C₆)alkyl group,
- Rₐ and Rₑ each represent, independently of each other, a hydrogen atom, a halogen atom, a (C₁-C₂₀)alkyl, (C₁-C₂₀)haloalkyl, aryl, heteroaryl, aryl-(C₁-C₆)alkyl, cycloalkyl, heterocycle, heterocycle-(C₁-C₆)alkyl, OR₂₂ or SR₂₃ group, and
- R_{b}, R_{c} and R_{d} each represent, independently of each other, a hydrogen atom, a halogen atom or a (C₁-C₂₀)alkyl, (C₁-C₂₀)haloalkyl, cycloalkyl, heterocycle or O(C₁-C₂₀)alkyl group;
▪ **R₁** represents a halogen atom, a (C₁-C₂₀)alkyl, (C₂-C₆)alkynyl, (C₁-C₂₀)haloalkyl, aryl, heteroaryl, aryl-(C₁-C₆)alkyl, heteroaryl-(C₁-C₆)alkyl, cycloalkyl, cycloalkyl-(C₁-C₆)alkyl, heterocycle, heterocycle-(C₁-C₆)alkyl, (CH₂)ₘOR₂₄, (CH₂)_{m'}SR₂₅, OR₂₆, SR₂₇, NR₂₈R₂₉, COR₃₂, OCOR₃₆ or NR₃₈COR₃₉ group, wherein m and m' are, independently of each other, equal to 1, 2 or 3, preferably 1;
▪ **R₄** represents a halogen atom, a (C₁-C₂₀)alkyl, (C₂-C₆)alkynyl, (C₁-C₂₀)haloalkyl, aryl, heteroaryl, aryl-(C₁-C₆)alkyl, heteroaryl-(C₁-C₆)alkyl, cycloalkyl, cycloalkyl-(C₁-C₆)alkyl, heterocycle, heterocycle-(C₁-C₆)alkyl, (CH₂)ₘOR₂₄, (CH₂)_{m'}SR₂₅, OR₂₆, SR₂₇, NR₂₈R₂₉, COR₃₂, OCOR₃₆ or NR₃₈COR₃₉ group, wherein m and m' are, independently of each other, equal to 1, 2 or 3, preferably 1;
or
both R₄ groups form together a bond or a chain selected from the group consisting of-C(R₄₂R₄₃)-, -(CH₂)ₙ-, -Si(R₄₄R₄₅)-, -CH₂-Y-CH₂-, and -Y-CH₂-CH₂-Y'-, wherein:
- Y and Y' each represent, independently of each other, O, S or NH,
- n is equal to 2 or 3,
- R₄₂ and R₄₃, each represent, independently of each other, a (C₁-C₆)alkyl or an aryl group, and
- R₄₄ and R₄₅ each represent, independently of each other, a (C₁-C₆)alkyl or an aryl group;
▪ **L_{X}** represents a bond, or a group selected from the group consisting of: 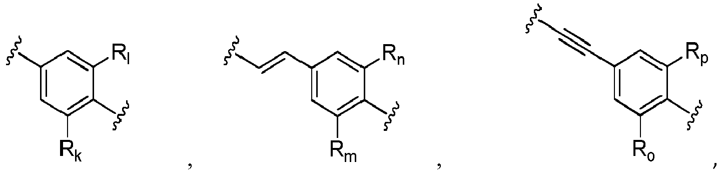 and 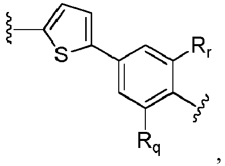
wherein Rₖ, Rₗ, Rₘ, Rₙ, Rₒ, Rₚ, R_{q} and Rᵣ each represent, independently of each other, a halogen atom, a (C₁-C₆)alkyl, (C₁-C₆)haloalkyl, aryl, heteroaryl, cycloalkyl, heterocycle, OR₄₈, SR₄₉ or NR₅₀R₅₁ group ;
and
**R₂** and **R₃** each represent, independently of each other, a hydrogen atom, a halogen atom, a (C₁-C₆)alkyl, (C₁-C₆)haloalkyl, (C₂-C₆)alkenyl, (C₂-C₆)alkynyl, aryl, heteroaryl, cycloalkyl, heterocycle, OR₅₆, SR₅₇ or NR₅₈R₅₉ group ;
with the proviso that:
- when L_{X} represents a bond, R₂ and R₃ do not represent a hydrogen atom, and
- when L_{X} does not represent a bond, R₂ and R₃ both represent a hydrogen atom;
or
**L_{X}** represents: 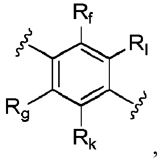 and R_{f} and R₂, and R_{g} and R₃ form together with the carbon atoms that carry them a cycloalkenyl or aryl group, wherein Rₖ and R₁ are as defined above;
▪ R₅ to R₈, R₁₁ to R₁₅, R₂₄, R₂₅, R₂₈, R₂₉, R₃₂, R₃₆, R₃₈, R₃₉, R₄₈ to R₅₁, and R₅₆ to R₅₉ each represent, independently of each other, a hydrogen atom, a (C₁-C₆)alkyl, (C₁-C₆)haloalkyl, aryl, heteroaryl, aryl-(C₁-C₆)alkyl, heteroaryl-(C₁-C₆)alkyl, cycloalkyl, cycloalkyl-(C₁-C₆)alkyl, heterocycle or heterocycle-(C₁-C₆)alkyl group, preferably a hydrogen atom or a (C₁-C₆)alkyl group, said group being optionally substituted by one or more groups selected from a halogen atom, a (C₁-C₆)alkyl, (C₁-C₆)haloalkyl, OR₆₄, SR₆₅ and NR₆₆R₆₇ group, wherein R₆₄ to R₆₇ each represent, independently of each other, a hydrogen atom or a (C₁-C₆)alkyl group,
with the proviso that R₅, R₆, R₄₈, R₄₉, R₅₆ and R₅₇ are not H;
▪ R₂₂ to R₂₃ and R₂₆ to R₂₇ each represent, independently of each other a (C₁-C₂₀)alkyl, notably (C₁-C₆)alkyl, (C₁-C₂₀)haloalkyl, notably (C₁-C₆)haloalkyl, aryl, heteroaryl, aryl-(C₁-C₆)alkyl, heteroaryl-(C₁-C₆)alkyl, cycloalkyl, cycloalkyl-(C₁-C₆)alkyl, heterocycle or heterocycle-(C₁-C₆)alkyl group, preferably a (C₁-C₂₀)alkyl, notably (C₁-C₆)alkyl group, said group being optionally substituted by one or more groups selected from a halogen atom, a (C₁-C₆)alkyl, (C₁-C₆)haloalkyl, OR₆₄, SR₆₅ and NR₆₆R₆₇ group, wherein R₆₄ to R₆₇ each represent, independently of each other, a hydrogen atom or a (C₁-C₆)alkyl group;
and
▪ A⁺ represents a monovalent, organic or inorganic cation.

2. The compound according to claim 1, wherein **X** represents an oxygen atom.

3. The compound according to claim 1 or 2, wherein **R₀** represents a hydrogen atom, a halogen atom, a (C₁-C₆)alkyl, (C₁-C₆)haloalkyl, cycloalkyl, heterocycle, OR₅, SR₆, NR₇R₈, CN or NO₂ group, or is selected from the group consisting of: 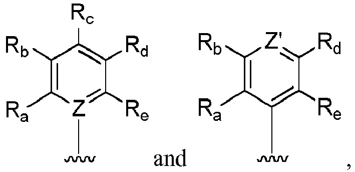 wherein:
- Z represents C or N⁺ A_{z}⁻ and Z' represents N or N⁺-R_{c}' A_{z}⁻, wherein
A_{z}⁻ represents a monovalent organic or inorganic anion, and
R_{c}' represents a (C₁-C₆)alkyl group,
- Rₐ and Rₑ each represent, independently of each other a hydrogen atom, a halogen atom, a (C₁-C₆)alkyl, (C₁-C₆)haloalkyl, OR₂₂ or SR₂₃ group, and
- R_{b}, R_{c} and R_{d} each represent, independently of each other, a hydrogen atom, a halogen atom or a O(C₁-C₂₀)alkyl, (C₁-C₆)alkyl or (C₁-C₆)haloalkyl group.

4. The compound according to claim 3, wherein **R₀** represents a hydrogen atom, a halogen atom, a (C₁-C₆)alkyl, (C₁-C₆)haloalkyl, OR₅, NR₇R₈, CN or NO₂ group, or is: 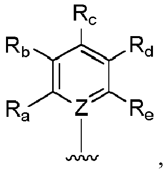 wherein:
- Z represents C,
- Rₐ and Rₑ each represent, independently of each other, a hydrogen atom, a halogen atom, a (C₁-C₆)alkyl, (C₁-C₆)haloalkyl or OR₂₂ group, and
- R_{b}, R_{c} and R_{d} each represent a hydrogen atom or a O(C₁-C₂₀)alkyl group, preferably R_{b}, R_{c} and R_{d} are the same,
preferably R₀ represents a hydrogen atom, a (C₁-C₆)alkyl, OR₅, NR₇R₈ or a CN group, notably a hydrogen atom, NR₇R₈ or a CN group.

5. The compound according to any one of claims 1 to 4, wherein **R₁** represents a halogen atom, a (C₁-C₆)alkyl, (C₁-C₆)haloalkyl, aryl, (CH₂)ₘOR₂₄, (CH₂)_{m'}SR₂₅, OR₂₆, SR₂₇ or NR₂₈R₂₉, group, wherein m and m' are, independently of each other, equal to 1, 2 or 3, preferably 1.

6. The compound according to any one of claims 1 to 5, wherein:
**R₄** represents a halogen atom, a (C₁-C₆)alkyl, (C₁-C₆)haloalkyl, aryl, (CH₂)ₘOR₂₄, (CH₂)_{m'}SR₂₅, OR₂₆, SR₂₇, or NR₂₈R₂₉, group, wherein m and m' are, independently of each other, equal to 1, 2 or 3, preferably 1;
or
both R₄ groups form together a bond or a chain selected from the group consisting of -C(R₄₂R₄₃)-, -(CH₂)ₙ-, -Si(R₄₄R₄₅)- and -Y-CH₂-CH₂-Y'-, wherein:
- Y and Y' each represent, independently of each other, O, S or NH,
- n is equal to 2 or 3,
- R₄₂ andR₄₃, each represent, independently of each other, a (C₁-C₆)alkyl group, and
- R₄₄ and R₄₅ each represent, independently of each other, a (C₁-C₆)alkyl group.

7. The compound according to any one of claims 1 to 6, wherein:
**L_{X}** represents a bond, or a group selected from the group consisting of: 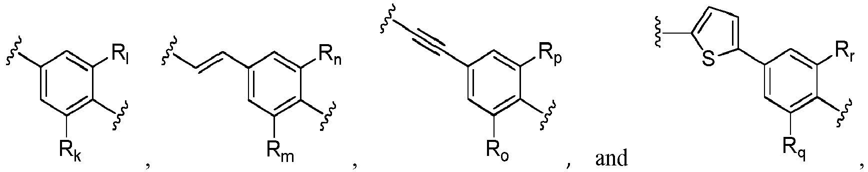
wherein Rₖ, Rₗ, Rₘ, Rₙ, Rₒ, Rₚ, R_{q} and Rᵣ each represent, independently of each other, a halogen atom, a (C₁-C₆)alkyl, (C₁-C₆)haloalkyl, aryl, heteroaryl, cycloalkyl or heterocycle group ;
and
**R₂** and **R₃** each represent, independently of each other, a hydrogen atom, a halogen atom, a (C₁-C₆)alkyl, (C₁-C₆)haloalkyl, aryl, heteroaryl, cycloalkyl, heterocycle, OR₅₆, SR₅₇, or NR₅₈R₅₉ group ;
with the proviso that:
- when L_{X} represents a bond, R₂ and R₃ do not represent a hydrogen atom, and
- when L_{X} does not represent a bond, R₂ and R₃ both represent a hydrogen atom.

8. The compound according to claim 7, wherein:
**L_{X}** represents a bond and **R₂** and **R₃** each represent, independently of each other, a halogen atom, a (C₁-C₆)alkyl, (C₁-C₆)haloalkyl, aryl, heteroaryl, OR₅₆, SR₅₇, or NR₅₈R₅₉group;
or **L_{X}** represents 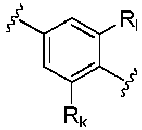
wherein Rₖ and Rₗ, each represent, independently of each other, a halogen atom, a (C₁-C₆)alkyl, (C₁-C₆)haloalkyl, an aryl or a heteroaryl group, and **R₂** and **R₃** both represent a hydrogen atom.

9. The compound according to any one of claims 1 to 8, wherein R₁ = R₄ and/or R₂ = R₃ and/or Rₐ = Rₑ and/or R_{b} = R_{c} = R_{d} and/or Rₖ = R₁ and/or Rₘ = Rₙ and/or Rₒ = Rₚ and/or R_{q} = R_{r.}

10. The compound according to any one of claims 1 to 9, wherein it is chosen from the following compounds:
| | |
|---|---|
| 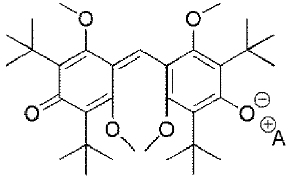 | 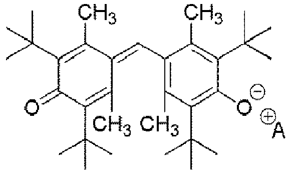 |
| **1** | **2** |
| 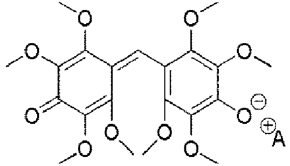 | 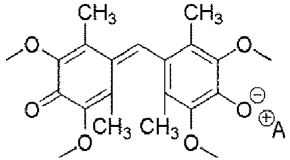 |
| **3** | **4** |
| 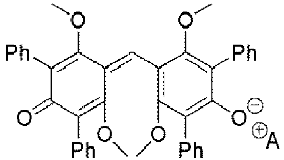 | 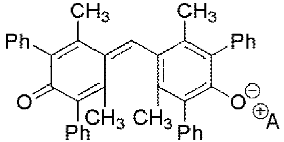 |
| **5** | **6** |
| 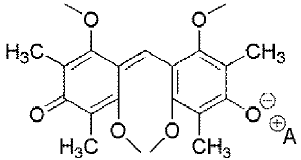 | 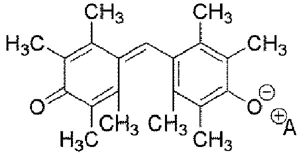 |
| **7** | **8** |
| 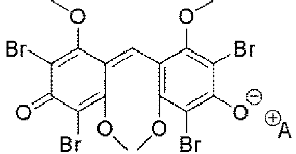 | 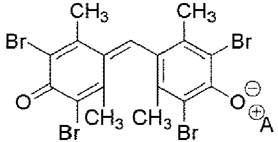 |
| **9** | **10** |
| 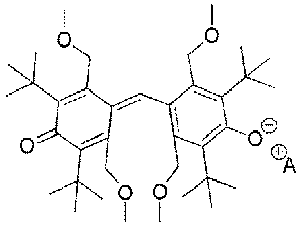 | 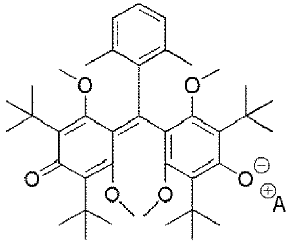 |
| **11** | **12** |
| 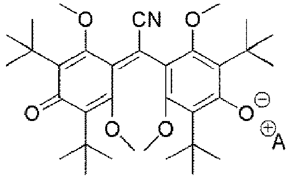 | 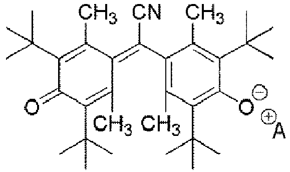 |
| **13** | **14** |
| 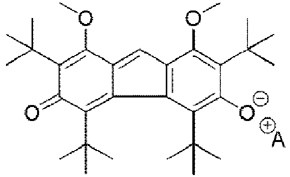 | 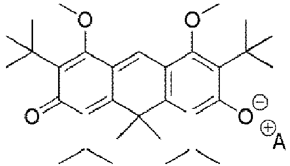 |
| **15** | **16** |
| 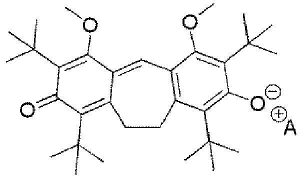 | 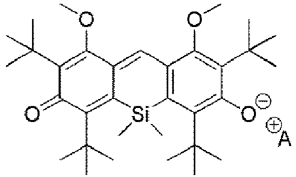 |
| **17** | **18** |
| 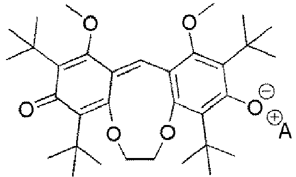 | 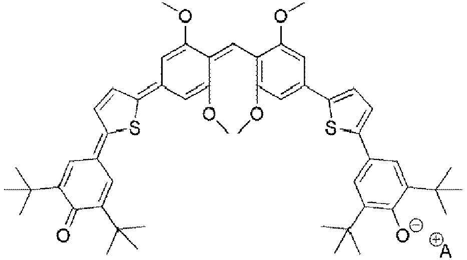 |
| **19** | **20** |
| 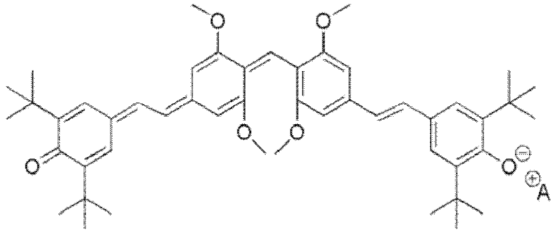 | |
| **21** | |
| 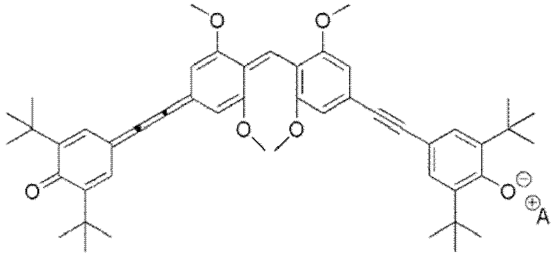 | |
| **22** | |
| 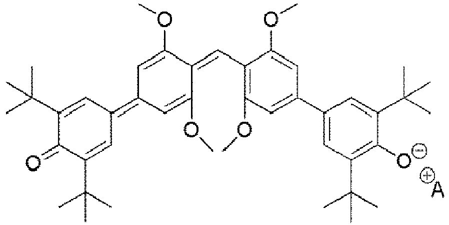 | 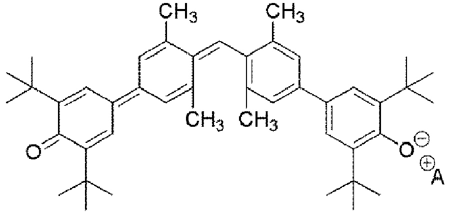 |
| **23** | **24** |
| 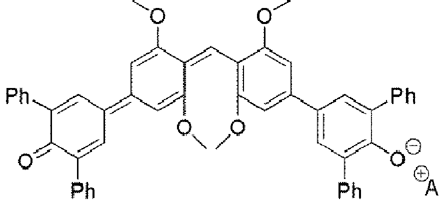 | 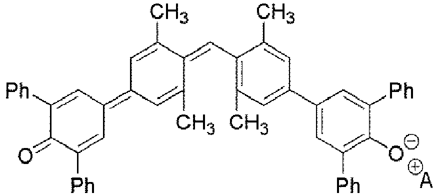 |
| **25** | **26** |
| 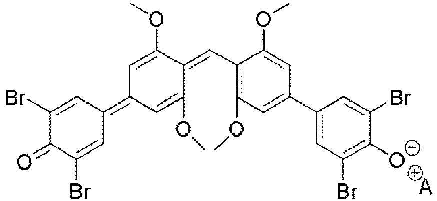 | 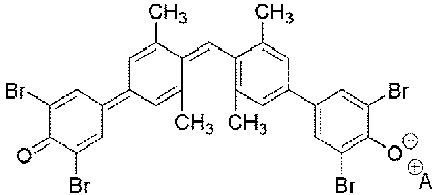 |
| **27** | **28** |
| 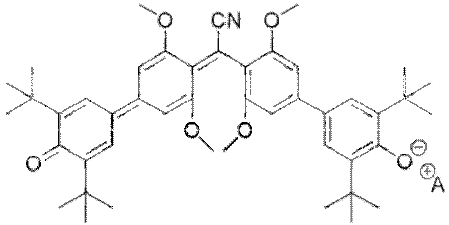 | 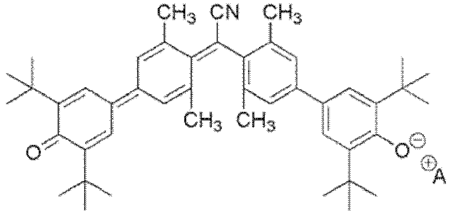 |
| **29** | **30** |
| 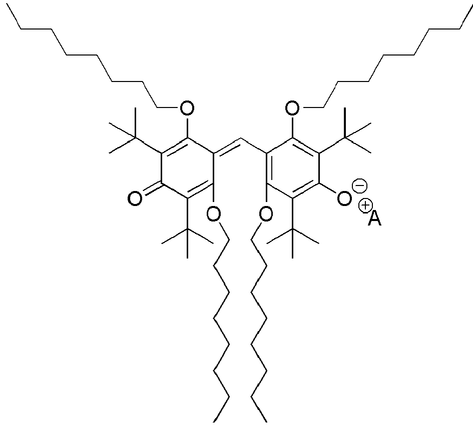 | 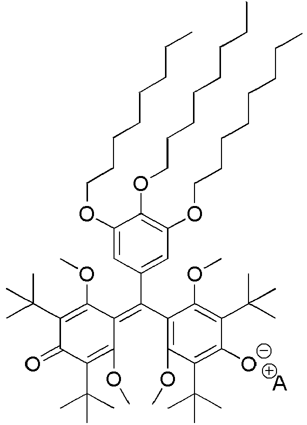 |
| **31** | **32** |
| 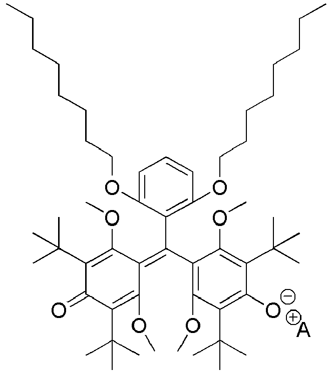 | 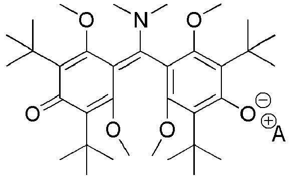 |
| **33** | **34** |
| 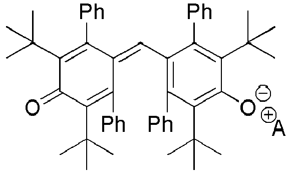 | |
| **35** | |

11. The compound according to any one of claims 1 to 10, wherein **A**⁺ represents an ammonium or a phosphonium ion or the conjugate acid of an organic nitrogen-containing bicyclic base.

12. The compound according to claim 11, wherein **A**⁺ is selected from the group consisting of tetrabutylammonium, tetrapropylammonium, tetraethylammonium, tetramethylammonium, benzyltrimethylammonium, phenyltrimethylammonium, hexadecylmethylammonium choline, triethylmethylammonium, diethyldimethylammonium, tetrabutylphosphonium, tetraphenylphosphonium, DBN-H⁺, DBU-H⁺, TBD-H⁺ and DABCO-H⁺, preferably from the group consisting of tetrabutylammonium, tetrapropylammonium, tetraethylammonium, tetramethylammonium, benzyltrimethylammonium, phenyltrimethylammonium, hexadecylmethylammonium choline, triethylmethylammonium and diethyldimethylammonium.

13. Use as dye or pigment of a compound as defined in any one of claims 1 to 12, notably as a luster pigment.

14. A reflective or photonic or nanophotonic or optoelectronic device, **characterized in that** it comprises at least one compound as defined in any one of claims 1 to 12.

15. A metal-like reflective coating or an organic-based metal-like liquid film, **characterized in that** it comprises at least one compound as defined in any one of claims 1 to 12.

16. A compound of following general formula (**Iᵢₙₜ**): 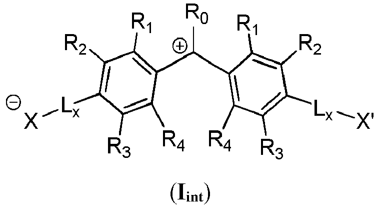 wherein:
▪ X represents an oxygen or a sulfur atom, and:
- when X represents an oxygen atom, X' represents OH, and
- when X represents a sulfur atom, X' represents SH; and
▪ R₀ to R₄ and L_{X} are as defined in any one of claims 1 to 10.

17. Use as dye or pigment of a compound according to claim 16.
